(19) Europäisches Patentamt
European Patent Office
Office européen des brevets

(11) Publication number: 0 513 703 A2

(12) EUROPEAN PATENT APPLICATION

(21) Application number: 92107866.3

(22) Date of filing: 11.05.92

(51) Int. Cl.[5]: C07D 487/04, C07D 519/00, A61K 31/40, //(C07D487/04, 209:00,209:00),(C07D519/00, 487:00,487:00)

(30) Priority: 13.05.91 IT MI911298
15.05.91 IT MI911335
27.05.91 IT MI911444
23.07.91 IT MI912027
18.12.91 IT MI913398
31.12.91 IT MI913512

(43) Date of publication of application: 19.11.92 Bulletin 92/47

(84) Designated Contracting States: AT BE CH DE DK ES FR GB GR IT LI LU MC NL PT SE

(71) Applicant: MAGIS FARMACEUTICI S.p.A.
Via Cacciamali 34/36/38
I-25128 Brescia(IT)

(72) Inventor: Puricelli, Laura
Via Taramelli, 7
I-20125 Brescia(IT)

(74) Representative: Gervasi, Gemma, Dr. et al
NOTARBARTOLO & GERVASI Srl 33, Viale Bianca Maria
I-20122 Milano(IT)

(54) **Carbamate esters of eseroline having anticholinesterase activity, a process for their preparation and relative pharmaceutical compositions containing them as the active principle.**

(57) Carbamate esters of eseroline having anticholinesterase activity characterized by the following formula (I):

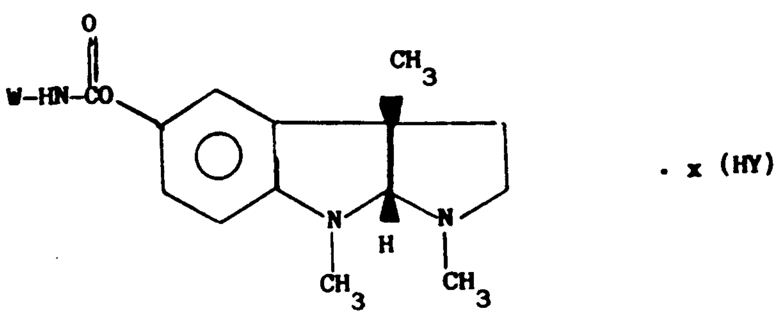

wherein W is a substituent, as defined in the following Specification, HY is a pharmaceutically acceptable organic or inorganic acid x may be 0, 1, 2 and 3 in case of monofunctional acids provided that x is 3 only when W is

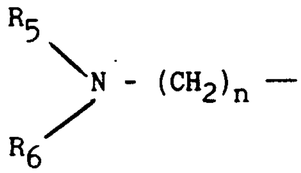

EP 0 513 703 A2

or 1/2 and 1/3 in case of bifunctional or trifunctional acids, a process for their preparation and pharmaceutical compositions containing said compounds , as the active principles, for the treatment of diseases associated with cholinergic disorders.

## FIELD OF THE INVENTION

The present invention relates to eseroline derivatives, having anticholinesterase activity, a process for their preparation and pharmaceutical compositions containing them as the active ingredient.

## PRIOR ART

Anticholinesterase activity of physostigmine has been known for years, but this compound has the drawback of being very toxic of having a short duration of action, and of exhibiting harmful side-effects of considerable gravity.

## THE PRESENT INVENTION

The present invetion relates to eseroline derivatives, namely to carbamate esters of eseroline, having anticholinesterase activity of formula (I) :

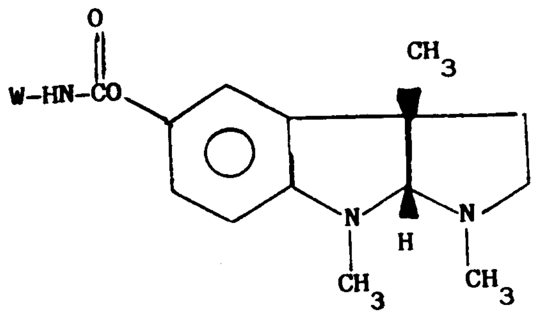

. x (HY)

wherein W is a substituent belonging to one of the following classes:

A)

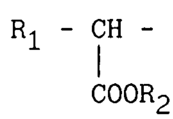

wherein $R_1$ is H, $-CH(CH_3)_2$, $-CH_2CH(CH_3)_2$,

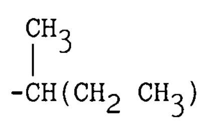

and $R_2$ is H or a $C_1$-$C_{20}$ alkyl radical

B) - A $-COOR_3$

wherein A is selected from a $C_2$ - $C_8$ linear or branched alkyl radical optionally containing one or more ethylenic unsaturations, a $C_2$ - $C_8$ linear or branched ketoalkyl radical, a $C_2$-$C_8$ linear or branched hydroxyalkyl radical optionally esterified with an organic acid

$R_3$ is H or a $C_1$ - $C_{20}$ alkyl radical optionally containing one or more ethylenic unsaturations provided that when $R_3$ = H, A must be different from ketoalkyl;

C) $R_4OM$

wherein $R_4$ is H, or $CH_3$-$(L)_m$-CO- wherein L is a bivalent linear or branched $C_1$-$C_{20}$ alkyl radical, optionally containing one or more ethylenic unsaturations; m is 0 or 1

M is a $C_1$-$C_{20}$ bivalent linear or branched alkyl radical optionally substituted with an aryl radical or an alicyclic radical; a bivalent alicyclic $C_3$-$C_7$ radical, or a bivalent aryl radical;

D)

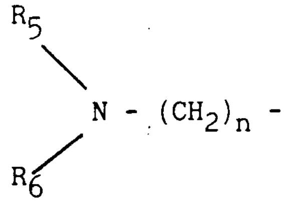

wherein $R_5$ and $R_6$ equal or different from eachother are selected from H, a $C_1$-$C_{20}$ alkyl radical, n is an integer comprised between 1 and 20

E)

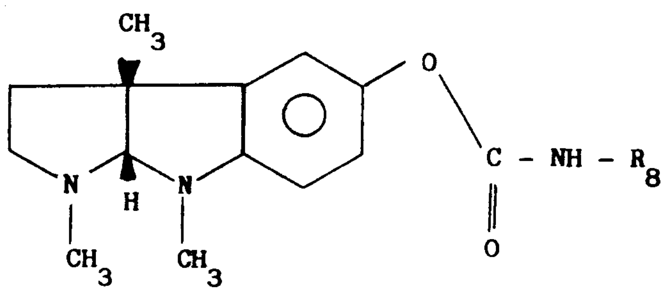

wherein $R_8$ is selected from the group consisting of:

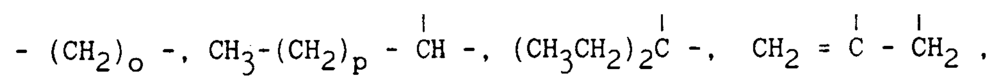

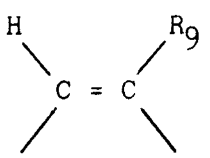

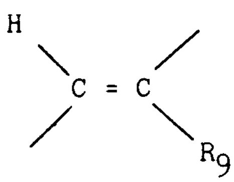

wherein o is an integer comprised between 1 and 8, p is an integer comprised between 1 and 3, $R_9$ is H or $CH_3$

F)

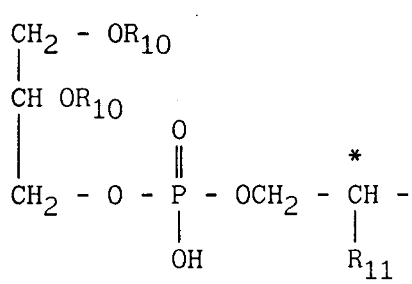

wherein $R_{11}$ is H or COOH, $R_{10}$ is H or a $C_2$-$C_{20}$ acyl radical, the carbon atom indicated with asterisk when $R_{11}$ is COOH is an asymmetric carbon and is characterized by having L, D or (D,L) configuration HY is a pharmaceutically acceptable organic or inorganic acid x may be 0, 1, 2 and 3 in case of monofunctional acids provided that x is 3 only when W is

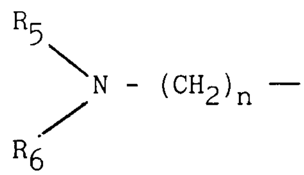

or 1/2 and 1/3 in case of respectively bifunctional or trifunctional acids.

The Applicant has in fact unexpectedly found that these compounds, while having an evident anticholinesterase activity, both tested "in vitro" on human pseudocholinesterase, and on acetylcholinesterase coming from Electrophorus Electricus, and "in vivo" on mouse, contemporaneously show a remarkably lower acute toxicity, and a longer action duration than physostigmine.

The compounds according to the present invention are therefore particularly useful in the therapeutic field for the treatment of various diseases associated with cholinergic disorders, such as Alzheimer disease, myasthenia gravis, glaucoma, and several pathologies of different neuromuscular origin .

The present invention further relates to therapeutical compositions containing as the active principle one or more of the eseroline derivatives of formula (I) in combination with suitable excipients or diluents for the treatment of diseases associated with cholinergic disorders, and for the treatment of memory dysfunctions caused by a cholinergic function decrease.

The therapeutical compositions according to the present invention are preferably in the form of oral or parenteral compositions.

The Applicant has also unexpectedly found that the compounds of general formula (I) of the present invention having the substituent W belonging to the above mentioned class C and particularly having W selected from the class consisting of: $R_4OM$ wherein $R_4$ and M have the above mentioned meanings, show also analgesic activity, and they are therefore useful as active principles for the preparation of pharmaceutical composition having analgesic activity for the pain treatment.

The present invention further relates to a process for preparing the above mentioned eseroline derivatives of formula (I), which in particular comprises the following steps:

a) reacting the eseroline of formula (II)

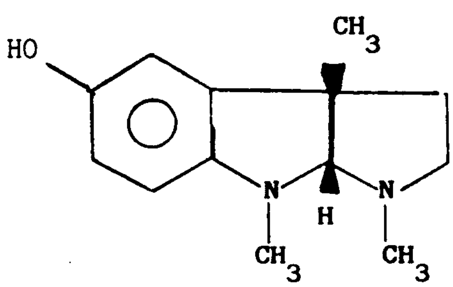

(II)

with the isocyanate of formula (III)

$W' - (N = C = O)_r$

wherein r is 1 or 2, W' is a substituent belonging to one of the following classes

A')

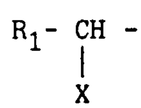

wherein $R_1$ has the above mentioned meaning

X is Cl or V wherein V is a protecting group of the -COOH function, preferably -COOBenzyl

B') $R'_3OOC - A' -$

wherein $R'_3$ is $PhCH_2$ or $= R_3$ but different from H and $A' = A$ but different from hydroxy- alkyl

C') MT

wherein M has the above mentioned meanings and T is a protecting group of the hydroxy function and r is 1.

D')

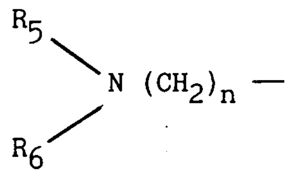

wherein $R'_5$ and $R'_6$ have the same meanings as $R_5$ and $R_6$ when they are both different from H, when $R_5$ or $R_6$ in the compound of formula (I)is H, $R'_5$ or $R'_6$ is G wherein G is a protecting group of the amino function such as carbobenzyloxy (CBZ), and **t**Butoxycarbonyl (BOC), or they are = O in case in the final compound $R_5 = R_6 = H$ n has the above mentioned meanings;

E') W' = $R_8$ wherein $R_8$ has the above mentioned meanings

F')

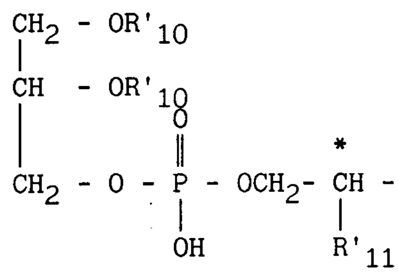

wherein $R'_{11} = R_{11}$ in case this is = H

and $R'_{11}$ = V wherein V is a protecting group of the -COOH function preferebly -COOBenzyl in case $R'_{11}$ = COOH

b) The compounds obtained in step (a) is treated with a stoichiometric amount of HY in a polar solvent to obtain the derivative of formula (I) having x = O.

## DETAILED DESCRIPTION OF THE INVENTION

In the derivatives according to formula (I) HY is preferably: tartaric acid, citric acid, salicylic acid, maleic acid, hydrochloric acid, phosphoric acid, sulforic acid.

The preferred eseroline derivatives having the substituent W belonging to class A are the following having:

a) $R_1$ is

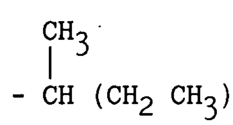

$R_2$ is H or n-heptyl

and x = O

a') $R_1$ is

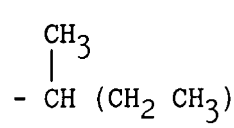

$R_2$ is H x = 1

and HY is a natural tartaric acid

a'') $R_1$ is $-CH_2\ CH\ (CH_3)_2$ $R_2$ is H and x = O
a''') $R_1$ is $CH(CH_3)_2$ $R_2$ is H and x = O
a$^{iv}$) $R_1$ and $R_2$ are H and x is O

The preferred eseroline derivatives having the substituent W belonging to class B are the following, in which:

b') A is $-(CH_2)_2$ - and $R_3$ is H or $-C_9H_{19}$ x = O
b'') A is $-(CH_2)_8-$ $R_3$ is H x = O or = 1 and in this case HY is tartaric acid
b''') A is $-CH_2CH(CH_3)$ and $R_3$ is H x is O
b$^{iv}$) A is $-COCH_2-$, $R_3$ is $-CH_3$ and x = O
b$^{v}$) A is $-CH(OH)CH_2-$ and $R_3$ is H and x = O
b$^{vi}$) A is $-CH(CH_2OH)-$ $R_3$ is H and x = O
b$^{vii}$) A is $-CH_2\ (CHOH)\ CH_2-$ $R_3$ is H
b$^{viii}$) A is $-CH_2\ CH_2CH(OH)CH_2-$
$R_3$ is H and x = O

The preferred eseroline derivatives having the substituent W belonging to class C are the following in which:

c) $R_4$ is H, M is

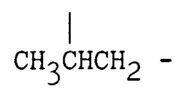

x = O
c') $R_4$ is $CH_3(CH_2)_7 - CH = CH - (CH_2)_7$ CO,
M is

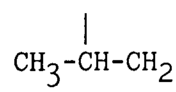

x = O
c'') $R_4$ is H, M is

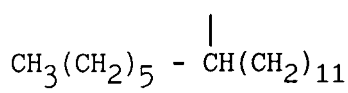

x = O
c''') $R_4$ is H, M is

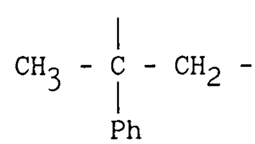

x = O
c$^{iv}$) $R_4$ is H or $CH_3\ (CH_2)_{16}$ - CO - M is

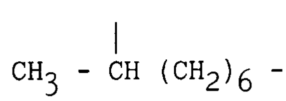

x = O
c$^{v}$) $R_4$ is H,

$$M = CH_3 - \overset{|}{C}H(CH_2)_6 -$$

x = O

$c^{vi}$) $R_4$ = H

M = 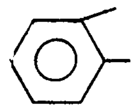

$c^{vii}$) $R_4$ = H

M = 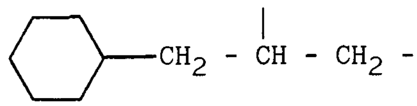

x = O

$c^{viii}$) $R_4$ = H,

$$M = CH_3 - \overset{|}{C}H - CH_2 -$$

x = 1

HY = tartaric acid

The preferred eseroline derivatives having the substituent W belonging to class D are the following in which:

d) $R_5$ = H, $R_6$ = methyl n is 7, x is O

d') $R_5$ is H, $R_6$ is methyl, n is 5, x is O

d'') $R_5$ and $R_6$ are n-heptyl, n is 4 , x is O

d''') $R_5$ is H, $R_6$ is n-heptyl, n is 4, x is O

$d^{iv}$) $R_5$ and $R_6$ are H, n is 7 and x is O

$d^{v}$) $R_5$ is H, $R_6$ is methyl, n is 7, x is 1 , HA is salicylic acid.

The preferred eseroline derivatives having the substituent W belonging to class E are the following in which:

e) $R_8$ is $(-CH_2)_o$- and o is 1,2,3,4,5,6,7 or 8 and x is O.

e') $R_8$ is $-(CH_2)_o$- o is 8, x is = 2 and HY is salicylic acid

e'') $R_8$ is

$$CH_3-(CH_2)_p - \overset{|}{C}H -$$

p is 3 and x is O

e''') $R_8$ is

$$CH_2 = \overset{|}{C} - \overset{|}{C}H_2$$

and x is O

$e^{iv}$) $R_8$ is

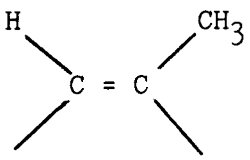

and x is O

$e^{v}$) $R_8$ is

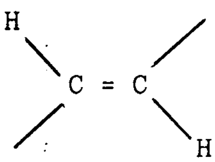

and x is O

$e^{vi}$) $R_8$ is

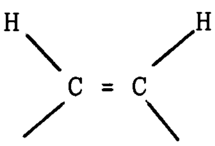

and x is O

The preferred eseroline derivatives having the substituent W belonging to class F are the following in which:

f) $R_{10}$ is $-COCH_3$, $R_{11}$ is H and x is O

f') $R_{10}$ and $R_{11}$ are H and x is O

f'') $R_{10}$ is $COCH_3$, $R_{11}$ is COOH and x is O

f''') $R_{10}$ is $COCH_3$, $R_{11}$ is H, x is 1 and HY is tartaric acid.

When in the above mentioned compounds of formula (I) having the substituent W which belongs to class A, $R_1$ is different from H, the carbon indicated with an asterisk is asymmetric, and it may present the following configuration: L, D, or (D,L), preferably it shows the L configuration.

When in the above mentioned eseroline derivatives of formula (I) having the substituent W belonging to class F, $R_{11}$ is COOH, the asymmetric carbon indicated with an asterisk, has L configuration.

The therapeutical compositions according to the present invention preferably contain the the derivatives of formula (I) in amounts ranging from 1 to 40 mg per unitary dose when in said compound the substituent W belongs to classes A, B, C, D or F).

While the therapeutical compositions according to the present invention preferably contain the derivative of formula (I) having substituent W belonging to class (E) in amounts ranging from 0.8 to 20 mg.

The eseroline of formula (II) used as the reactant in step (a) in the process according to the present invention is obtained as described in Brossi et al Heterocycles 26 pages 1271, 1987. Preferably step (a) is carried out in an aromatic solvent at room temperature for a time ranging from 1 to 6 hours and step (b) is carried out in absolute ethanol.

The process for preparing the eseroline derivatives of formula (I) having W belonging to class A is further characterized in that: in step a) the isocyanate of formula (II) is used having r = 1 and W' belonging to class A') and it also comprises between step (a) and (b) the following steps:

i) when substituent X contained in W' = Cl, the compound obtained in step (a) is treated with magnesium, then with carbon dioxide and finally with an inorganic acid

or

i') when X = V = -COO-Benzyl the compound coming from step (a) is treated with hydrogen at atmospheric pressure in a mixture of an alcohol and an aprotic solvent in the presence of a hydrogenation catalyst

ii) when compound (I) is to be obtained having $R_2$ different from H, the compound coming from step (a) , step (i), or (i') is treated in an organic solvent with a stoichiometric amount of alcohol $R_2OH$ wherein $R_2$

is as described above in the presence of a condensing agent and of a basic catalyst. Preferably said step (i) is performed by using 1 to 1.5 moles of magnesium per mole, in an anhydrous aromatic solvent, at the boiling temperature of the solvent for a time ranging from 0.5 to 3 hours and the resulting reaction mixture is then treated with an at least stoichiometric amount of an inorganic acid or step (i') is carried out by using Pt/C as the catalyst in a mixture of toluene and ethanol and in step (ii) the solvent is tetrahydrofurane, the condensing agent is dicyclohexylcarbodiimide, the basic catalyst is dimethylaminopyridine

The process for preparing the eseroline derivatives of formula (I) having W belonging to class B is further characterized in that: when in step (a) the isocyanate of formula (III) is used having r = 1 and W' = $PhCH_2OCO$-A'-
A' has the above mentioned meanings, the intermediate (IA) is obtained

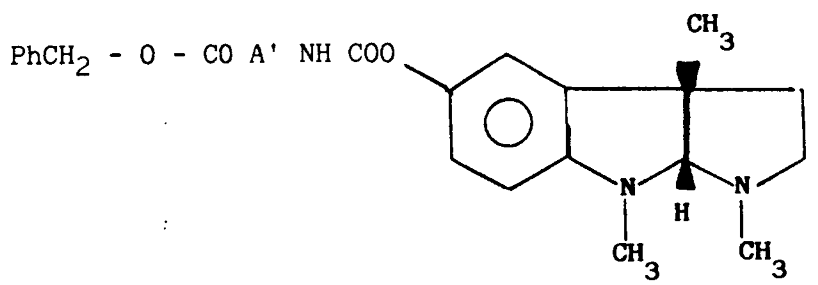

(IA)

and the process further comprises the following steps between steps (a) and (b):

i) when the compound of formula (I) to be obtained presents $R_3$ = H, the carbamate of formula (IA) is treated with hydrogen at ambient temperature and at atmospheric pressure in a mixture of alcohol/aprotic solvent in ratio ranging from 1:9 to 1:0.5 respectively in the presence of a hydrogenation catalyst until it is completely converted into the derivative of formula (I) in which $R_3$ is H and A has the above mentioned meanings provided that it is different from ketoalkyl group;

ii) the product obtained in step (i) is esterified by treatment with an alcohol $R_3OH$ in which $R_3$ has the above mentioned meanings in the presence of a condensing agent and optionally a basic catalyst: in an aprotic solvent at a temperature ranging from 15 to 40 °C, thereby obtaining the derivative of formula (I) wherein $R_3$, is different from H, and A is different from ketoalkyl. When $R'_3$ in W' is different from H, the process according to the present invention consists only of the above mentioned steps (a) and (b).

With said process starting from the isocyanate of formula (III) having W' in which $R'_3$ is different from H and A' = ketoalkyl it is possible to obtain the compound of formula (I) in which W has $R_3$ different from H and A is a ketoalkyl group; while starting from the isocyanate of formula (III) in which W' shows $R'_3$ = $-OCH_2$-Ph, together with steps (a), (i) and (ii) it is possible to obtain the derivatives having A = hydroxyalkyl.

The process for preparing the eseroline derivatives of formula (I) having W belonging to class C is further characterized in that:
when in step (a) the isocyanate of formula (III) has r = 1 and W' belonging to class C' it further comprises the following steps between steps (a) and (b):

i) the protecting group T of the hydroxyl function is removed

ii) when the compound of formula (I) to be obtained presents $R_4$ = $CH_3$ $(L)_m$ - CO -, the compound obtained in step (i) is reacted with the corresponding acid of formula

$CH_3$ $(L_m)$ COOH (IV)

wherein L and m have the above mentioned meanings.

In this case the protecting group T is $-OCH_2$ - Ph and step (i) is realized by carrying out a hydrogenation reaction in the presence of $PtO_2$ at atmospheric pressure in a mixture of ethanol and benzene at room temperature; and step (ii) is carried out in the presence of a stoichiometric amount of dicyclohexylcarbodiimide optionally in the presence of diethylaminopyridine in chloroform.

The process for preparing the eseroline derivatives of formula (I) having W belonging to class D is further characterized in that:
W' in the isocyanate of formula (III) belongs to class D' and r = 1 it further comprises between step (a) and (b) the following step:

(i) when the compound of formula (I) to be obtained presents W having $R_5$ or $R_6$ = H, the product coming from step (a) in which W' brings $R'_5$ or $R'_6$ = G, wherein G has the above mentioned meanings, is treated with hydrogen at atmospheric pressure in the presence of a mixture of an aprotic solvent and alcohol in the presence of a hydrogenation catalyst;

or

(i') when the compound of formula (I) to be obtained presents W having $R_5$ = $R_6$ = H, the product coming from step (a) having W' wherein $R'_5$ = $R'_6$ = O is treated with $H_2$ by using as the catalyst Pt/C at atmospheric pressure.

The process for preparing the eseroline derivatives of formula (I) having W belonging to class E is characterized in that: in step (a) the isocyanate of formula (III) is used having W' belonging to class (E') and r = 2.

The process for preparing the eseroline derivatives of formula (I) having W belonging to class F is characterized in that:

in step (a) the isocyanate of formula (III) is used having W' belonging to class (F') and r = 1 it comprises between step (a) and (b) the following steps:

(i) when the compound to be obtained of formula (I) presents $R_{10}$ = H, it further comprises the following step among (a) and (b):

i) the product coming from step (a) is treated with an alkaline metal alkoxide in catalytic amounts in alcohol, and the product obtained is recovered after neutralization of the alkaline alkoxide with a mineral acid, followed by salification with an inorganic base;

and/or

i') when in the compound to be obtained $R_{11}$ = COOH the product coming from step (a) having $R'_{11}$ = V = -COOBenzyl the compound is treated with hydrogen at atmospheric pressure in a mixture of an alcohol and an aprotic solvent in presence of a hydrogenation catalyst.

In this case in step (i) methanol or absolute ethanol is preferably used as the solvent, and sodium or potassium ethylate, as the alkaline metal alkoxide.

The isocyanate of general formula (III) is prepared according to the followind steps:

a) $W'\text{-}(COOH)_r$

in which W' belongs to class (A'), (B'), (C'), (D'), (E') and r has the above mentioned meanings, is reacted with thionyl chloride, thereby obtaining the acid chloride having the following formula (VI)

$W'\text{-}(COCl)_r$ (VI)

b) the organic acid chloride is reacted with sodium azide at a temperature ranging from 5 to 25 °C, for a time comprised between 0.5 hours and 3 hours in water optionally in the presence of a water miscible solvent, the aqueous phase is removed and an aromatic solvent is added, perferably benzene, preheated at 40-70 °C and after a time period of 0.5 -3 hours the isocyanate of formula (III) is recovered.

When W' belongs to class (A') and X is -COObenzyl the isocyanate of formula (III) is obtained in the following way starting from benzylester of the aminoacid of formula (VII)

$$R_1 - \underset{\displaystyle COO\,Benzyl}{\underset{|}{CH}} - NH_2 \qquad (VII)$$

wherein $R_1$ has the above mentioned meanings which is reacted with phosgene in toluene or chloroform.

For preparing the isocyanate of formula III in which W' belongs to class (E') the glycerophosphorylox-yaminoderivative of formula (VIII)

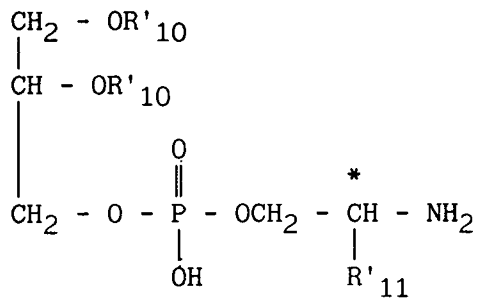

in which $R'_{10}$ and $R'_{11}$ have the above mentioned meanings is reacted with phosgene in carbon tetrachloride.

The following examples are reported for illustrative, but not limitative purposes.

Preparation of the carbamate esters having W belonging to class (A)

**Example 1** Preparation of (3aS-cis) 1, 2, 3, 3a,8,8a hexahydro-1,3a, 8-trimethyl-pyrrol (2,3 -b) indol-5-ol-L-(3-methyl-2 carboxy)butyl-carbamate ester [Derivative in which $R_1$ is

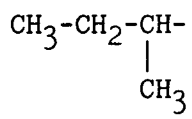

and $R_2$ = H (MGS101)] 21.8 g of eseroline, obtained according to Brossi et al. (Meterocycles et al 26,5 g, 1271, 1987) in 500 ml of benzene are slowly added to a solution under stirring, composed by 32 g of L-2-isocyano-3-methyl-pentanoate of the benzyl derivative of L-isoleucine (isocyanate of formula (III) having W' = $R_1$-CH(X)-wherein X = -COO-$CH_2$-Ph, $R_1$ has the above mentioned meaning), while maintaining the reaction temperature lower than 20 °C. When the addition is terminated, the reaction is left under stirring at room temperature for 3 hours .

The benzene is evaporated at reduced pressure and the residue is dissolved in chloroform, it is loaded on a silica gel column and eluted with a mixture chloroform -methanol: 98:2.

The fraction containing the carbamate is separated and the solvent is evaporated to dryness at reduced pressure .

Obtained are 27.09 g of a white solid having molecular weight 465

| Elemental Analysis | C | H | N |
|---|---|---|---|
| Calculated (%) | 69.68 | 7.53 | 9.03 |
| Found (%) | 69.91 | 7.34 | 8.77 |

10 g of the carbamate thus obtained are dissolved in tetrahydrofurane and hydrogenated at atmospheric pressure, utilizing 0.8 g of 5% Pt/C as the catalyst. When the reaction is completed, the mixture is filtered on celite, and it is evaporated to dryness under reduced pressure. Obtained are 7.6 g of a white solid, having M.W. 375.4.

| Elemental analysis | C | H | N |
|---|---|---|---|
| Calculated (%) | 63.97 | 7.78 | 11.20 |
| Found (%) | 64.00 | 7.80 | 11.10 |

**Example 2** Alternative preparation of MGS (001)

0.1 moles eseroline are introduced in 2.5 l benzene containing in solution 0.2 moles L,1-chloro-1-isocyanate-2-methyl-butane (isocyanate of formula (III) having X = Cl).

The mixture is left under stirring for 3 hours. The benzene is evaporated, the residue is dissolved in 100 ml of chloroform.

The solution is transferred in a silicic acid column (1.5 kg) and eluted with chloroform-methanol mixture (98:2).

The fraction containing eseroline L,1-(1-chloro-2-methylbutanol) carbamate ester is separated and evaporated to dryness.

Thus 21.28 g of product are obtained, with a 58% yield and a 99% titre.

| Elemental analysis | C | H | N | Cl |
|---|---|---|---|---|
| Calculated (%) | 62.38 | 7.66 | 11.49 | 9.71 |
| Found (%) | 61.0 | 7.5 | 11.50 | 9.70 |

m.w. = 365.5

2.68 g of magnesium shavings are introduced into a 200 ml three-neck flask equipped with stirrer, condenser and separatory funnel.

The shavings are covered with 5 ml anhydrous ether.

One iodine crystal and 2 g of the previously obtained carbamate ester are added.

The reaction is left to proceed for 30 minutes. Then 5 ml anhydrous ethyl ether are added and, in 30 minutes, an anhydrous ethyl ether solution containing further 32.464 g of carbamate ester.

A violent reflux is prevented by outside cooling of the reactor in a water bath.

The reaction is left to proceed for 90 minutes, then the mixture is cooled to -12°C.

Further 10 ml ether are added and carbon dioxide is bubbled into the mixture.

The flow rate is adjusted so that the temperature of the mixture is kept below -5°C.

After 90 minutes, the compound is hydrolyzed by adding 50 ml of a 25% water solution of sulphuric acid, cooling to 0°C.

The ether phase separates.

The water solution is extracted again with ether and neutralized with sodium hydroxide.

The precipitated product is filtered, washed with water and dissolved in 100 ml chloroform.

The solution is passed through a silicic acid column (1.5 kg) and eluted with the chloroform-methanol solution (98:2).

The fraction containing the product is evaporated to dryness.

21.3 g (yield 60.5%) are obtained, with a titre over 99%.

The spectral analyses confirm the structure.

**Example 3**

Preparation of (3aS-cis) 1, 2, 3, 3a,8,8a hexahydro-1,3a, 8-trimethyl-pyrrol (2,3 -b) indol-5-ol-L-(3-methyl-2 carboxy-n-heptyl)-butyl-carbamate [Derivative having $R_1$ is

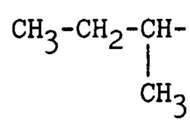

and $R_2$ = n-heptyl (MGS102)]

21.8 g of eseroline, in 500 ml of benzene are slowly added to a solution under stirring, composed by 32 g of n-heptyl L-2-isocyano-3-methyl-pentanoate of L-isoleucine (isocyanate of formula (III) having W' = $R_1$-CH(X)-wherein X = -COO-n-heptyl wherein $R_1$ has the above mentioned meaning), while maintaining the reaction temperature lower than 10°C

When the addition is terminated, the reaction is left under stirring at room temperature for 3 hours .

The solvent is evaporated under reduced pressure and the residue obtained is dissolved in the minimum volume of $CHCl_3$, this solution is the charged onto a silica gel column and eluted with a mixture of $CHCl_3$ :$CH_3OH$ 98:2.

The fraction containing the desired product is evaporated to dryness under reduced pressure, and 28.5 g of a white solid, are obtained. This product presents a M.W. of 473.65.

| Elemental Analysis | C | H | N |
|---|---|---|---|
| Calculated (%) | 68.46 | 9.15 | 8.87 |
| Found(%) | 68.50 | 9.20 | 8.80 |

**Example 4**

Preparation of tartrate of (3aS-cis) 1, 2, 3, 3a,8,8a hexahydro-1,3a, 8-trimethyl-pyrrol (2,3 -b) indol-5-ol-L-(3-methyl-2carboxy-n-heptyl)-butyl-carbamate [Derivative having $R_1$ is

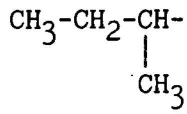

and $R_2$ = n-heptyl, HY = tartaric acid, x = 1.]

0.1 moles (3 as-cis) 1,2,3,3a,8,8a-hexahydro 1,3a,8,trimethyl pyrrol [2-3-b] indol 5-ol-L-(3-methyl-2-carboxy n-heptyl)-butyl carbamate ester, prepared as described in Example 3 and 0.1 moles of tartaric acid are dissolved in 100 ml of absolute ethanol. The solvent is evaporated by the aid of vacuum, the residue is brought to dryness under 0.01 mmHg.

**Example 5 -** Preparation of of 2-isocyano-3-methyl-pentanoate of benzyl isocyanate (III) used in Example 1.

23.1 g of N-Boc L-isoleucine are dissolved in acetonitrile and under stirring at room temperature and 4 g of NaOH (0.1mol) dissolved in 20 ml of water .

When the mixture is homogeneous 3.4 g (10 mmol) of tetrabutylammonium bisulfate and 20 g (0.120 mol) of benzylbromide are added.

The mixture is heated to the solvent reflux temperature under strirring for 3 hours, and then concentrated to a small volume under reduced pressure.

The reaction is then cooled to room temperature and water and chloroform are added. Two phase separate, the organic layer is washed twice with 5% $NaHCO_3$, then with water, and finally dried on anhydrous sodium sulfate, filtered and evaporated to dryness. Hexane is added to the residue obtained, and the precipitate obtained is filtered. Obtained are 25.5 g of N-Boc L-isoleucine-benzylester with a yield of about 80%. The instrumental confirm the structure.

The product thus obtained, by treatment with trifluoroacetic acid and thiophenol at 20 °C for 60 minutes brings to the formation of L-isoleucine-benzylester, which is then isolated by treatment of aqueous NaOH and successive extraction.

Obtained are 15.5 g of anhydrous product which is then dissolved in $CHCl_3$, cooled to 0-5 °C and slowly dropped in equimolar amounts of phosgene dissolved in toluene. When the addition is completed, the mixture is heated to 45 °C for 20', while maintaining a strong stirring.

After evaporation to dryness obtained are 17 g of benzyl 2-isocyano-3-methyl-pentanoate.

The corresponding heptyl 2-isocyano -3-methyl-pentanoate is obtained with the same method, by using n-heptyl-bromide in place of benzylbromide.

**Example 6** Preparation of L-1-chloro-1-isocyanate-2-methyl butane (isocyanate of formula (III) in which W' is $R_1$-CH(X) and X is Cl.

In a 1-liter flask, equipped with stirrer and thermometer and dipped in a water bath, 46 g (0.7 moles) sodium azide in 150 ml water are introduced.

A solution of L-2-chloro-3-methyl valeroyl chloride (75.7 g 0.4 moles) in 150 ml of acetone is added slowly under stirring keeping the temperature between 10 and 15 °C.

The mixture is left under stirring at this temperature for 1 hour.

The lower water phase is separated and the upper phase is put rapidly into 500 ml benzene, heated to 60°C.

Nitrogen develops rapidly To remove the last traces of nitrogen, the mixture is heated to 60-70°C until no further nitrogen develops. The conversion of the azide to isocyanate requires about 1 h.

The solution is filtered and benzene removed by distillation.

The distillation of the residue yields approximately 60 g isocyanate (yield about 85%).

**Example 7**

Preparation of (3-aS-cis) 1,2,3,3a,8,8a-hexahydro 1,3a,8-trimethyl pyrrol [2,3-b]-indol-5-ol-L-2(4-methylvaleric acid) carbamate ester[carbamate ester having $R_1$ is

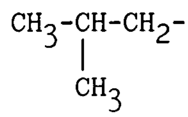

and $R_2$ = H (MGS 103)].

It is prepared similarly to example no. 1, starting from the isocyanate of formula (III) having X = $COOCH_2Ph$ and $R_1$ having the above mentioned meanings.

The instrumental and elemental analyses confirm the structure.

**Example 8**

Preparation of (3-as-cis) 1,2,3,3a,8,8a-hexahydro 1,3a,8-trimethyl pyrrol [2,3-b]-indol-5-ol-L-(3-methyl butyric acid) carbamate ester [carbamate ester having $R_1$ is

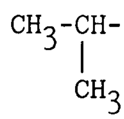

$R_2$ is H (MGS 104)].

It is prepared similarly to example 1, starting from the isocyanate of formula (III) , wherein $R_1$ has the above mentioned meanings and X= $COOCH_2Ph$, and eseroline

The instrumental and elemental analyses confirm the structure.

**Example 9**

(3-as-cis) 1,2,3,3a,8,8a-hexahydro 1,3a,8-trimethyl pyrrol [2,3-b]-indol-5-ol (acetic acid) carbamate ester. [carbamate ester having $R_1$ = H, $R_2$ = H (MGS 105)]

It is prepared similarly to example 2, starting from eseroline and the isocyanate having X = Cl, and $R_1$ = H.

The instrumental and elemental analyses confirm the structure.

Preparation of the carbamate esters having W belonging to class (B)

**Example 10**

Preparation of the eseroline derivative of formula (I) wherein A is $-(CH_2)_2-$ and $R_3$ is H [M001]

2.183 g of eseroline are introduced into a flask fitted with stirrer and reflux condenser ; a solution of 1.15 g of the isocyanate of formula (III) wherein A' is $-(CH_2)_2-$ and $R'_3$ is $PhCH_2-$ in 200 ml of benzene is added; the mixture thus obtained is maintained under slow stirring for 3 hours.

The benzene is then evaporated and 80 ml of petroleum ether followed by 40 ml of hydrochloric acid

0.1 M are added to the residue.

The solvent is then removed and the aqueous phase is extracted three times with ethyl ether after neutralizing the acid solution with sodium bicarbonate.

The ethereal extracts are then collected and dried on anhydrous sodium sulfate.

The solvent is evaporated and the residue thus obtained is dissolved in 10 ml of a mixture of chloroform and methanol in the volumetric ratio 98:2.

The solution is eluted on a chromatographic silica gel column, utilizing as the eluent the same mixture of chloroform and methanol previously used for solubilizing the residue.

The solvent of the fraction containing M001 is evaporated by the aid of vacuum.

20 ml of a mixture toluene ethanol in volumetric ratio 1/1 are added to the residue obtained, and said mixture is then treated with hydrogen at atmospheric pressure, using as the catalyst Pd/C at 10% . At the end of the reaction the catalyst is removed by filtration and the filtrate is completely evaporated.

The residue obtained is then dissolved in 10 ml of benzene: by adding heptane a white crystalline precipitate is obtained consisting of M001 product (2.5 g ; 75% yield)

The spectral analyses confirm the assigned structure.

| Elemental Analysis | C | H | N |
|---|---|---|---|
| Calculated (%) | 61.25 | 6.91 | 12.61 |
| Found (%) | 61.3 | 7.0 | 12.54 |

M.W.333

**Example 11**

Preparation of the eseroline derivative of formula (I) wherein A is $-CH_2CH(CH_3)-$ and $R_3$ is H [M002].

This product is prepared with the same operating conditions described in Example 1, starting from eseroline and the isocyanate of formula (III) wherein A' is $-CH_2CH(CH_3)-$ and $R'_3$ is $PhCH_2-$.

The spectral and elemental analyses confirm the assigned structure.

**Example 12**

Preparation of the eseroline derivative of formula (I) wherein A is $-(CH_2)_8$ and $R_3$ is H [M003].

This product is prepared with the same operating conditions described in Example 1, starting from eseroline and the isocyanate of formula (III) wherein A' is $-(CH_2)_8$ and $R'_3$ is $PhCH_2-$.

The spectral and elemental analyses confirm the assigned structure.

**Example 13**

Preparation of the eseroline derivative of formula (I) wherein A is $-COCH_2-$ and $R_3$ is $CH_3$ [M004].

The same operating conditions described in example 10 are followed starting from eseroline and the isocyanate of formula (III) wherein A' is $-COCH_2-$ and $R'_3$ is $CH_3$ up to the separation of the desired product on chromatographic column of silica gel.

The solvent of the fraction containing the product M004 is is then evaporated by the aid of vacuum, the residue thus obtained is dissolved in benzene, containing heptane: the precipitation of the M004 product is then observed. The spectral and elemental analyses confirm the assigned structure.

**Example 14**

Preparation of the eseroline derivative of formula (I) wherein A is $-CH(OH)CH_2$ and $R_3$ is H [M005].

This product is prepared with the same operating conditions described in Example 10, starting from eseroline and the isocyanate of formula (III) wherein A' is $-COCH_2-$ and $R'_3$ is $PhCH_2-$.

The spectral and elemental analyses confirm the assigned structure.

**Example 15**

Preparation of the isocyanate of formula (III) wherein A' is $-(CH_2)_2-$ and $R'_3$ is $Ph-CH_2-$.

6.57 g (1 mol) of sodium azide dissolved in 150 ml of water are charged into a 1 litre flask, fitted with a

stirrer and a thermometer dipped in an ice bath.

To the solution thus obtained, after cooling to about 10 ° C, a solution of 150 ml of acetone containing 0.5 mol of benzylsuccinylchloride is added, which was previously prepared by esterifying only one carboxylic group of succinic acid with benzyl alcohol, and subsequently treating the product obtained with thionylchloride in 150 ml of acetone.

The mixture is maintained under stirring for 1 hour at a temperature comprised between 10 and 15 ° C.

The organic phase is separated from the aqueous phase, and it is quickly transferred into 500 ml of benzene previously heated at 60 ° C. Nitrogen gas quickly forms.

The solution is left at 60-70 ° C until nitrogen gas ceases developing.

The transformation of azide into isocyanate requires about 1 hour. The solution is then filtered, the benzene is removed by distillation and the residue is dissolved in 20 ml of a mixture chloroform / methanol 98/2.

The solution is then loaded onto a chromatographic silica gel column, by eluting with the same solvent mixture used for preparing the residue solution.

The fraction containing the desired isocyanate is separated, concentrated to a small volume, then heptane is added, the isocyanate precipitate is observed, which is recovered by filtration and dried under vacuum at 40 ° C. The spectral analyses confirm the assigned structure.

| Elemental Analysis | C | H | N |
|---|---|---|---|
| Calculated (%) | 64.39 | 8.33 | 10.61 |
| Found (%) | 64.03 | 8.02 | 10.59 |

M.W. 205

**Example 16**

Preparation of the eseroline derivative of formula (I) wherein A is $-(CH_2)_2-$ and $R_3$ is $n-C_9H_{19}$.

3.33 g of the eseroline derivative of formula (I) wherein A is $-(CH_2)_2-$ and $R_3$ is H (M001) are dissolved in 100 ml of chloroform together with 2.06 g of dicyclohexylcarbodiimide, 1.44 g of n-nonylic alcohol and 0.1 g of diethylaminopyridine.

After 6 hours at room temperature the mixture is filtered in order to remove the dicyclohexylurea formed, the solution obtained is washed with water and the chloroform phase is separated and dried on anhydrous sodium sulphate.

The solvent is evaporated to dryness and the residue is washed with 5 ml of acetone.

The product thus purified is dried under vacuum at 40 ° C.

Obtained are 4.5 g of pure product (yield about 98%).

The spectral and elemental analyses confirm the assigned structure.

**Example 17**

Preparation of the tartrate of the eseroline derivative of formula (I) wherein A is $-(CH_2)_8-$ and $R_3$ is H .

0.1 mol of the compound obtained in Example 12 [M003] and 0.1 mol of tartaric acid are dissolved in 100 ml of anhydrous ethanol.

After the solution is formed the solvent is evaporated by the aid of vacuum, thereby obtaining the desired salt.

The spectral and elemental analyses confirm the assigned structure.

**Example 18**

Preparation of the eseroline derivative of of formula (I) wherein A is $-CH(CH_2OH)-$ and $R_3$ is H [M006]

Operating as described in example 10, starting from eseroline and the isocyanate of formula (III) wherein A' is -CH(CHO)- and $R'_3$ is $Ph-CH_2-$, the derivative M006 is obtained.

The spectral and elemental analyses confirm the assigned structure.

**Example 19**

Preparation of the eseroline derivative of formula (I) wherein A is $-CH_2(CHOH)CH_2-$ and $R_3$ is H [M007]

Operating as described in example 10, starting from eseroline and the isocyanate of formula (III) wherein A' is $-CH_2COCH_2-$ and $R'_3$ is $Ph-CH_2-$, the derivative M007 is obtained.

The spectral and elemental analyses confirm the assigned structure.

**Example 20**

Preparation of the eseroline derivative of formula (I) wherein A is $-CH(CH_3)CH_2-$ and $R_3$ is H [M008]

Operating as described in example 10, starting from eseroline and the isocyanate of formula (III) wherein A' is $-CH(CH_3)CH_2-$ and $R_1$ is $Ph-CH_2-$, the derivative M008 is obtained.

The spectral and elemental analyses confirm the assigned structure.

**Example 21**

Preparation of the eseroline derivative of formula (I) wherein A is $-CH_2CH_2(CHOH)CH_2-$ and $R_3$ is H [M009]

Operating as described in example 1, starting from eseroline and the isocyanate of formula (III) wherein A' is $-CH_2CH_2COCH_2-$ and $R'_3$ is $Ph-CH_2-$, the derivative M009 is obtained.

The spectral and elemental analyses confirm the assigned structure.

Preparation of the carbamate esters having W belonging to class (C)

**Example 22**

Preparation of trimethyl -1,3a,8 hexahydro-1,2,3a,8,8a-pyrrol(2,3-b)indol-5(3aS,8aR)2-hydroxypropylcarbamate [derivative of formula (I) wherein $R_4$ = H,

$$M = CH_3-\overset{|}{CH}-CH_2-,$$

x = O, (ES001)]

2.183 g of eseroline are placed into a flask fitted with a stirrer and a reflux condenser, 1.91 g of 2-benzyloxypropylisocyanate dissolved in 200 ml of benzene are added; the mixture thus obtained is left under slow stirring for 3 hours.

The benzene is then evaporated and 80 ml of petroleum ether are added to the residue, followed by 40 ml of 0.01 mol of hydrochloric acid.

The solvent is removed and the residue is extracted with ethyl ether, after neutralization of the acid solution with sodium bicarbonate.

The ethereal extracts are collected and dried on anhydrous sodium sulfate . The solvent is evaporated and the residue is dissolved in 10 ml of a mixture chloroform methanol respectively in a volumetric ratio 98:2.

The solution is eluted on a chromatographic silica gel column, utilizing as the eluent a mixture of chloroform and methanol in the same above mentioned volumetric ratio.

The solvent of the fraction containing ES001 is evaporated by the aid of vacuum. 10 ml of a mixture benzene/ethanol in ratio 1:1 are added and a hydrogenation reaction is carried out at atmospheric pressure, by using as the catalyst $PtO_2$, the catalyst is removed by filtration, and the filtrate is evaporated to dryness by the aid of vacuum.

The residue is dissolved in 10 ml of benzene, and by adding heptane a pure white crystalline precipitate is obtained: 2.4 g (75% yield).

The spectral analyses confirm the structure.

| Elemental Analysis | C | H | N |
|---|---|---|---|
| Calculated (%) | 63.93 | 7.89 | 13.156 |
| Found (%) | 64 | 7.89 | 13.01 |

M.W.319.397

**Example 23**

Preparation of trimethyl -1,3a,8 hexahydro-1,2,3a,8,8a-pyrrol(2,3-b)indol-5(3aS,8aR)-2-oleoxypropylcarbamate [derivative of formula (I) wherein $R_4$ = $CH_3(CH_2)_7$-CH=CH--$(CH_2)_7$CO-,

M = 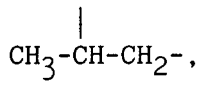

x = O, (ES002)]

3.194 g of the compound obtained in example 22 together with 2.824 g of oleic acid, 2.06 g of dicyclohexylcarbodiimide and 0.1 g of dimethylaminopyridine are dissolved in 50 ml of anhydrous chloroform.

The mixture is left to react for 6 hours, then the dicyclohexylurea formed is filtered, the chloroform solution is washed with 200 ml of hydrochloric acid 0.01 N. The chloroform phase is dried on anhydrous sodium sulfate.

The solvent is evaporated and the residue washed with acetone.

Obtained are 5.8 g of ES002, the instrumental and elemental analyses confirm the structure.

**Example 24**

Preparation of trimethyl -1,3a,8-hexahydro-1,2,3a,8,8a-pyrrol(2,3-b)indol-5-(3aS,8aR)12-hydroxystearylpropylcarbamate [derivative of formula (I) wherein $R_4$ = H,

M = 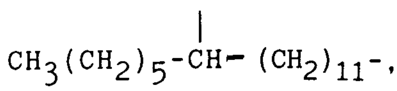

x = O, (ES003)].

The compound ES003 is prepared, by following the same working conditions described in example 22, starting from eseroline and 12-benzyloxy-stearylisocyanate.

**Example 25**

Preparation of trimethyl -1,3a,8, hexahydro-1,2, 3a, 8, 8a-pyrrol (2,3-b) indol-5 (3aS,8aR)-2-hydroxy-2-phenylpropylcarbamate
[derivative of formula (I) wherein $R_4$ = H,

M = 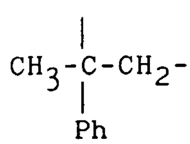

x = O, (ES004)]

The compound ES004 is prepared, by following the same working conditions described in example 22, starting from eseroline and 2-benzyloxy-2-phenyl-propylisocyanate.

**Example 26**

Preparation of trimethyl -1,3a,8 hexahydro-1,2,3a,8, 8a-pyrrol(2,3-b)indol-5(3aS,8aR)-2-hydroxy-phenyl-carbamate [derivative of formula (I) wherein $R_4$ = H,

M= 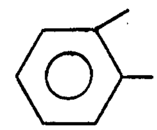

x = O, (ES005)]

The compound ES005 is prepared, by following the same working conditions described in example 22 , starting from eseroline and 2-benzyloxy-phenylisocyanate.

**Example 27**

Preparation of trimethyl -1,3a,8 hexahydro-1,2,3a,8,8a-pyrrol(2,3-b)indol-5-(3aS,8aR)-2-hydroxy-octanylcarbamate (ES006).
[derivative of formula (I) wherein $R_4$ = H,

M = $CH_3-CH-(CH_2)_6-$,

x = O, (ES006)];
The compound ES006 is prepared, by following the same working conditions described in example 22, starting from eseroline and 2-benzyloxy-octanylisocyanate.

**Example 28**

Preparation of trimethyl -1,3a,8 hexahydro-1,2,3a,8,8a-pyrrol(2,3-b)indol-5-(3aS,8aR)-2-stearoyloxyoctanylcarbamate [derivative of formula (I) wherein $R_4$ = $CH_3(CH_2)_{16}$-CO-

M = $CH_3-CH-(CH_2)_6-$,

x = O, (ES007)]

The compound ES007 is prepared, by following the same working conditions described in example 23, using as the reactants the compound obtained in example 27 (ES006) and stearic acid.

**Example 29**

Preparation of trimethyl -1,3a,8, hexahydro-1,2,3a,8,-8a pyrrol(2,3-b)indol-5-(3aS,8aR)-3-cyclohexyl-2-hydroxypropylcarbamate [derivative of formula (I) wherein $R_4$ = H,

M = 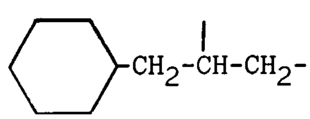

x = O, (ES008)];
The compound ES008 is prepared, by following the same working conditions described in example 22, starting from eseroline and 2-benzyloxy-3-cyclohexylpropylisocyanate.

**Example 30**

Preparation of tartrate of trimethyl -1,3a,8 hexahydro-1,2,3a,8,8a-pyrrol(2,3-b)indol-5(3aS,8aR)2-hydroxypropylcarbamate
[derivative of formula (I) wherein $R_4$ = H,

$$M = CH_3\text{-}\overset{|}{C}H\text{-}CH_2\text{-},$$

x = 1, HY = tartaric acid (ES009)]

0.1 mol of the compound obtained in example 22 (ES001) and 0.1 mol of tartaric acid are dissolved in 100 ml of absolute ethanol.

When the solution is formed the solvent is evaporated by the aid of vacuum.

**Example 31**

Preparation of 2-benzyloxypropylisocyanate [intermediate of formula (III) wherein

$$M = CH_3\overset{|}{C}H\text{-}CH_2\text{-}$$

T = - Obenzyl used for preparing the derivative ES001, which was prepared as described in Example 22]

6.57 g (1 mol) of sodium azide in 150 ml of water are loaded into a 1 litre flask fitted with a stirrer and a thermometer dipped in an ice bath.

A solution of 2-benzyloxybutanoylchloride (65 g) in 150 ml of acetone is added to the solution previously cooled to 10 ° C.

The mixture is stirred for 1 hour while maintaining the temperature between 10 and 15 ° C.

The organic phase is quickly transferred into 500 ml of benzene previously brought to 60 ° C.

Nitrogen gas quickly forms. In order to remove all the nitrogen the solution is brought to 60-70 ° C and maintained at this temperature as long as no nitrogen gas development is observed. The azide transformation into the isocyanate requires about 1 hour.The solution is filtered, the benzene is removed by distillation, the residue is dissolved in 20 ml of a chloroform-methanol solution ( 98:2).

The solution is eluted on a silica chromatographic silica gel column, utilizing as the eluent a mixture of chloroform: methanol (98:2).

The fraction containing the isocyanate is separated and concentrated , heptane is added a white precipitate is obtained which is then recovered and dried in an oven at 40 ° C under vacuum. The instrumental and elemental analyses confirm the structure.

<u>Eseroline derivatives of formula (I) in which W belongs to class D</u>

**Example 32 -** (3a S-*cis*) - 1,2,3,3a,8,8a hexahydro - 1,3a,8 - trimethyl pyrrol [2,3-b]-indol-5-ol-N-methylaminoheptyl carbamate ester [derivative (I) in which $R_5$ is H, $R_6$ is methyl, n is 7, and x is O](MGF 001).

21.83 g of eseroline were placed in 2.5 litres of benzene containing in solution 30.5 g of N-CBZ-methylamino heptyl isocyanate [compound (III) in which $R'_5$ is -G = -CBZ, $R'_6$ is methyl, and n is 7] prepared as described in example 38, and were agitated for 3 hours.

The benzene was then evaporated and the residue was dissolved in 60 ml of chloroform.

The solution was placed in a silicic acid column (1.5 kg), and eluted with a 98:2 chloroform-methanol mixture.

The fraction containing the carbamate was separated and brought to dryness.

Approximately 40.5 grams of the product were obtained, which is dissolved in tetrahydrofurane and hydrogenated under atmospheric pressure, using 2.5 grams of 5 % Pt/C, as the catalyst. When the reaction

was completed, it was filtered under vacuum and the filtrate was evaporated to dryness.

Obtained were about 22.5 grams of the above mentioned product, which is then crystallized from a mixture of toluene and hexane. The instrumental analyses confirmed its structure.

| Elemental Analysis: | C | H | N |
|---|---|---|---|
| calculated (%) | 68.04 | 9.27 | 14.43 |
| found (%) | 68 | 9.2 | 14.4 |

m.w. 388

**Example 33 -** (3a S-*cis*) - 1,2,3,3a,8,8a hexahydro - 1,3a,8 - trimethyl pyrrol [2,3,b]-indol-5-ol-N-methyl-aminopentyl carbamate ester [derivative (I) in which $R_5$ is H, $R_6$ is methyl, n is 5, and x is O](MGF 002).

The process was the same as that described in example 32, starting with eserolin and N-CBZ-methylamino pentyl isocyanate [compound (III) in which $R'_5$ is -G is -CBZ, $R'_6$ is methyl, and n is 5].

**Example 34 -** (3a S-*cis*) - 1,2,3,3a,8,8a hexahydro - 1,3a,8 - trimethyl pyrrol [2,3,b]-indol-5-ol-N,N-diheptylamino butyl carbamate ester [derivative (I) in which $R'_5$ and $R'_6$ are n-heptyl, n is 4, and x is O]-(MGF 003).

The process was the same as that described in example 32, starting with eserolin and N,N-diheptylamino butyl isocyanate [compound (III) in which $R'_5$ and $R'_6$ are n-heptyl, and n is 4], but obviously in this case the hydrogenation is not carried out.

**Example 35 -** (3a S-*cis*) - 1,2,3,3a,8,8a hexahydro - 1,3a,8 - trimethyl pyrrol [2,3,b]-indol-5-ol-N-heptylamino butyl carbamate ester [derivative (I) in which $R_5$ is H, $R_6$ is n-heptyl, n is 4, and x is O](MGF 004).

The process was the same as that described in example 32, starting with eserolin and N-CBZ-heptylamino butyl isocyanate [compound (III) in which $R'_5$ is -G = -CBZ, $R'_6$ is n-heptyl, and n is 4].

**Example 36 -** (3a S-*cis*) - 1,2,3,3a,8,8a hexahydro - 1,3a,8 - trimethyl pyrrol [2,3,b]-indol-5-ol-amino heptyl carbamate ester [derivative (I) in which $R_5$ and $R_6$ are H, n is 7, and x is O](MGF 005).

The process was the same as that described in example 32, starting with eserolin and amino heptyl isocyanate [compound (III) in which $R'_5$ and $R'_6$ are O, and n is 7], at the end of the reaction the nitro group was reduced by carrying out a hydrogenation reation under atmospheric pressure, using 5% Pt/C as the catalyst. The product MGF005 was obtained.

**Example 37 -** Salicylate of (3a S-*cis*) - 1,2,3,3a,8,8a hexahydro - 1,3a,8 - trimethyl pyrrol [2,3,b]-indol-5-ol-N-methyl-aminoheptyl carbamate ester [derivative (I) in which R is H, $R_1$ is methyl, n is 7, x is 1, and HA is salicylic acid].

Equimolar quantities of derivative MGF 001 prepared as described in example 1 and salicylic acid were dissolved in alcohol.

The alcohol was evaporated with the aid of vacuum, and the desired salt was obtained which may be purified by further crystallization.

**Example 38 -** N-methyl-aminoheptyl isocyanate [compound (III) in which $R'_5$ is CBZ, $R'_6$ is methyl, and n is 7].

80 g of sodium azide dissolved in 150 ml of water were introduced into a 2 litre flask with an agitator and a thermometer in an ice bath.

A mixture of 162.7 g (0.5 mol) of chlorhydrate of N-CBZ-methylamino octanoyl chloride and 200 ml of acetone was added to the resulting solution via a separator funnel whilst being strongly agitated and at sufficient speed to keep the temperature at from 0 ° C to 5 ° C.

The mixture was agitated at this temperature for 1 hour.

Agitation was stopped, the lower acqueous phase was separated, and the upper phase was slowly

added to 1500 ml of benzene preheated to a temperature of 60 ° C, the mixture was then heated to the reflux temperature, thus removing the last traces of water and Nitrogen gas quickly formed (approximately 90 minutes).

The solution was then filtered, and the benzene removed with the aid of a vacuum.

The resulting residue was purified by repeated precipitations from hexane, and about 114 g of isocyanate were obtained (yield approximately 75%).

| Elemental Analysis: | C | H | N |
|---|---|---|---|
| calculated (%) | 67.10 | 7.89 | 9.21 |
| found(%) | 66.85 | 7.99 | 9.50 |

m.w. 304

**Example 39 -**

(3a S-*cis*) - 1,2,3,3a,8,8a hexahydro - 1,3a,8 - trimethyl pyrrol [2,3-b]-indol-5-ol-1,8-octandicarbamate ester (MS-001).

43.66 g of eserolin were placed in 2.5 litres of benzene containing in solution 19.63 g of 1,8-octandiisocyanate prepared as described in example 40, and were stirred for 3 hours.

The benzene was evaporated and the residue was dissolved in 100 ml of chloroform.

The solution is passed a through silicic acid column (2.0 kg), and eluted with the chloroform-methanol (98:2) mixture.

The fraction containing the carbamate was separated and dried. 41.14 grams of carbamate were obtained with a minimum titre of 99% (Yield about 65%).

The product obtained is crystallized from toluene, thereby obtaining a whitish solid having melting point 135-137 ° C.

| Elemental Analysis: | C | H | N |
|---|---|---|---|
| calculated: | 68.32 | 8.28 | 13.28 |
| found(%): | 68.4 | 8.3 | 13.25 |

m.w. 632.86

**Example 40 -**

Preparation of 1,8-octandiisocyanate.

13.44 g (0.2 moles) of sodium aside in 42 ml of water were placed in a 1 litre flask with an agitator and a thermometer dripped in an ice bath.

A solution of 20 g of sebacic acid chloride in 50 ml of acetone was slowly added whilst maintaining the reaction mixture under agitation at a temperature of from 0 ° C to 5 ° C.

The mixture was stirred at this temperature for 1 hour.

The lower acqueous phase was separated, and the upper phase was quickly added to 500 ml of benzene which was previously heated to 60 ° C.

Nitrogen gas quickly formed, and in order to remove all traces of the gas, the mixture was further heated 60 ° C to 70 ° C, until no more nitrogen was formed. The transformation of the azide into isocyanate required about 1 hour.

The solution was then filtered, and the benzene removed by distillation.

The distillation of the residue provided 13.2 g of diisocyanate (yield approximately 80%).

| Elemental Analysis: | C | H | N |
|---|---|---|---|
| calculated: | 61.20 | 8.22 | 14.27 |
| found: | 61.3 | 8.25 | 14.2 |

m.w. 196.24

**Example 41 -**

Salicylate of (3a S-*cis*) - 1,2,3a,8,8a hexahydro - 1,3a,- trimethyl pyrrol [2,3-b]-indol-5-ol-1,8-octandicarbamate ester (MS-002).

0.1 moles of (3a S-*cis*) - 1,2,3a,8,8a hexahydro - 1,3a,8 - trimethyl pyrrol [2,3-b]-indol-5-ol-1,8-octandicarbamate ester were prepared as described in example 39, and dissolved in 1000 ml of absolute ethanol.

0.2 moles of salicylic acid were added, the mixture was heated to 40 ° C, and the solvent evaporated with the aid of a vacuum.

The product obtained was vacuum dried (0.01 mmHg), then crystallized from isopropanol in an inert environment. Obtained were 67 g of the desired product (yield about 85%).

Instrumental and elemental analyses confirmed its structure.

Using the procedure described in example 39, the following compounds were obtained:

(3a S-*cis*) - 1,2,3,3a,8,8a hexahydro - 1,3a,8 - trimethyl pyrrol [2,3-b]-indol-5-ol-methandicarbamate ester (MS-003);

(3a S-*cis*) - 1,2,3,3a,8,8a hexahydro - 1,3a,8 - trimethyl pyrrol [2,3-b]-indol-5-ol-1,2-ethandicarbamate ester (MS-004);

(3a S-*cis*) - 1,2,3,3a,8,8a hexahydro - 1,3a,8 - trimethyl pyrrol [2,3-b]-indol-5-ol-1,3-propandicarbamate ester (MS-005);

(3a S-*cis*) - 1,2,3,3a,8,8a hexahydro - 1,3a,8 - trimethyl pyrrol [2,3-b]-indol-5-ol-1,4-butandicarbamate ester (MS-006);

(3a S-*cis*) - 1,2,3,3a,8,8a hexahydro - 1,3a,8 - trimethyl pyrrol [2,3-b]-indol-5-ol-1,5-pentandicarbamate ester (MS-007);

(3a S-*cis*) - 1,2,3,3a,8,8a hexahydro - 1,3a,8 - trimethyl pyrrol [2,3-b]-indol-5-ol-1,1-pentandicarbamate ester (MS-008);

(3a S-*cis*) - 1,2,3,3a,8,8a hexahydro - 1,3a,8 - trimethyl pyrrol [2,3-b]-indol-5-ol-3,3-pentandicarbamate ester (MS-009);

(3a S-*cis*) - 1,2,3,3a,8,8a hexahydro - 1,3a,8 - trimethyl pyrrol [2,3-b]-indol-5-ol-1,6-hexandicarbamate ester (MS-010);

(3a S-*cis*) - 1,2,3,3a,8,8a hexahydro - 1,3a,8 - trimethyl pyrrol [2,3-b]-indol-5-ol-1,7-heptandicarbamate ester (MS-011);

(3a S-*cis*) - 1,2,3,3a,8,8a hexahydro - 1,3a,8 - trimethyl pyrrol [2,3-b]-indol-5-ol-*cis*-2-methyl-1,2-ethylendicarbamate ester (MS-012);

(3a S-*cis*) - 1,2,3,3a,8,8a hexahydro - 1,3a,8 - trimethyl pyrrol [2,3-b]-indol-5-ol-propylendicarbamate ester (MS-013);

(3a S-*cis*) - 1,2,3,3a,8,8a hexahydro - 1,3a,8 - trimethyl pyrrol [2,3-b]-indol-5-ol-*trans*-1,2-ethylendicarbamate ester (MS-014);

(3a S-*cis*) - 1;2,3,3a,8,8a hexahydro - 1,3a,8 - trimethyl pyrrol [2,3-b]-indol-5-ol-*cis*-1,2-ethylendicarbamate ester (MS-015);

Instrumental and elemental analyses confirmed the structures of the above listed compounds.

**Example 42 -** preparation of (3aS-cis) 1,2,3,3a,8,8a-hexahydro -1,3a,8 trimethyl-pyrrol-[2,3-b]-indol-5-ol-diacetylglycerylphosphoryl-ethyl carbamate ester [derivative of formula (I) wherein $R_{11}$ is H, $R_{10}$ is $-COCH_3$ and x is O] (MGR001).

21,93 g of eseroline are charged into 2.5 l of benzene containing in solution 35.22 g of diacetylglycerylphosphorylethylisocyanate, prepared as described later-on in example 46. The mixture is left under stirring at room temperature for 4 hours .

The solvent is evaporated, and the residue obtained is purified on a silicic acid column (1.5kg) by eluting with the solvent mixture chloroform methanol (98:2). The fraction containing the carbamate is separated and the solvent evaporated.

Obtained is the derivative (I) above indicated (g 33.5) having titre: about 99%.

The spectral analyses, and the elemental one, reported herinbelow, confirm the assigned structure.

| Elemental analysis | C | H | N | P |
|---|---|---|---|---|
| calculated (%) | 50.82 | 6.31 | 12.68 | 5.69 |
| Found (%) | 51.0 | 6.4 | 7.65 | 5.65 |

M.W. 543.5

**Example 43 -** (3aS-cis) 1,2,3,3a,8,8a-hexahydro -1,3a,8 trimethyl-pyrrol-[2,3-b]-indol-5-ol-glycerylphosphoryl-ethyl carbamate ester [derivative of formula (I) wherein $R_{11}$ is H, $R_{10}$ is H and n is O] (MGR002)

0.1 mol of (3aS-cis) 1,2,3,3a,8,8a-hexahydro -1,3a,8 trimethylpyrrol-[2,3-b]-indol-5-ol-diacetylglycerylphosphoryl-ethyl carbamate ester obtained as described in Example 42, are dissolved in 200 ml of absolute ethyl alcohol, containing 0.2 mmol of sodium methylate .

The mixture is left under stirring for 2 hours, filtered and evaporated. After adding chloroform to the residue, the mixture obtained is acidified with aqueous hydrochloric acid, and then neutralized with sodium bicarbonate, and finally filtered.

The chloroform phase is dried on sodium sulfate. A product is obtained with a titre about 99%.

The spectral and the elemental analysis confirm the assigned structure.

**Example 44** Preparation of (3aS-cis) 1,2,3,3a,8,8a-hexahydro - 1,3a,8 trimethyl-pyrrol-[2,3-b]-indol-5-ol-diacetyl-glycerylphosphoryl-2L -carboxylethyl carbamate ester [derivative of formula (I) wherein $R_{11}$ is COOH, $R_{10}$ is $-COCH_3$, the asymmetric carbon indicated with an asterisk presents L configuration, and n is O] (MGR003).

The same operating conditions are followed as those described in example 42, by using the isocyanate obtained from diacetylglycerylphosphoryl-L-serine benzylester as reported in example 47, which is then hydrogenated in the presence of a hydrogenation catalyst in order to hydrolize the benzylester.

Obtained are about 36 g of the desired carbamate of formula (I) having titre 99%.

The spectral and elemental analyses confirm the assigned structure.

**Example 45 -**Preparation of tartrate of (3aS-cis) 1,2,3,3a,8,8a-hexahydro -1,3a,8 trimethyl-pyrrol-[2,3-b]-indol-5-ol-diacetyl-glycerylphosphoryl-ethyl carbamate ester [derivative of formula (I) wherein $R_{11}$ is H, $R_{10}$ is $-COCH_3$ and x is 1 and HY is tartaric acid] .

0.1 moles of MGR001 derivative and 0.1 mol of tartaric acid are dissolved in 300 ml of ethanol. When the solution is formed, the solvent is evaporated by the aid of vacuum.

**Example 46 -** Diacetylglycerylphosphoryl-ethyl-isocyanate [isocyanate of formula (III) wherein $R_{11}$ is H and $R_{10}$ is $COCH_3$] 500 ml of anhydrous ethylacetate previously saturated with phosgene into a 5 l flask .

1.5 l of ethylacetate containing in solution 0.1 mol of diacetylglyceryphosphoryl ethanolamine . The ethyl acetate is distilled, 800 ml of preheated carbon tetrachloride are added to the residue and the insoluble residue is filtered.

Two third of carbon tetrachloride are evaporated, the solution is cooled and the isocyante formed is filtered. Obtained are 28 g of isocyanate (yield 86%).

The spectral and elemental analyses confirm the assigned structure.

**Example 47 -** Diacetylglycerylphosphoryl-2-L-carboxybenzylethyl isocyanate [isocyanate of formula (III) wherein $R'_{11}$ is COOBenzyl, $R_{10}$ is $-COCH_3$ and the carbon atom bringing the asterisk is asymmetric and has L configuration].

The same operating conditions described in Example 46 are followed, starting from phosgene and diacetylglycerylphosphoryl-L-serine benzylester.

The spectral and elemental analyses confirm the assigned structure.

We report hereinbelow the pharmacological and toxicological tests of the compounds of the invention. The compounds were compared with respect to physostigmine with ponderal values and from the various tests conducted it resulted evident that the derivatives according to the invention exhibit an improved

therapeutical index and a longer activity duration.

These values result better confirmed if they are converted into equivalent or molar values.

Pharmacological tests of the eseroline derivatives of formula (I) having the substituent W belonging to class A

The biological properties of some of the derivatives having the substituent W belonging to class A were compared with those of physostigmine.

Particularly the acute toxicity (LD50), the "in vivo" inhibition of cerebral and serumal acetylcholinesterase, the spontaneous motility, the antagonism against the central stimulating action of an anticholinergic agent (scopolamine), the electroencephalographic activity, and the effect on the curarization duration by succinylcholine.

Preliminary pharmacokinetics trials were also carried out.

**Acute toxicity**

The acute toxicity test was carried out on mice (Swiss strain of the average weight of 22 g (females) and 26 g (males) 5+5 animals per dose, period of observation 7 days).

The letal doses 50 (LD50) by oral route (suspension in 2 % carboxymethylcellulose) are reported herinbelow:

TABLE 1

| LD50 per os in the mouse of physostigmine and of the eseroline derivatives having the substituent W belonging to class A | |
|---|---|
| Physostigmine | 4.37 ± 1.22 mg/kg |
| MGS101 | 332.8 ± 6.20 mg/kg |
| MGS102 | 358.3 ± 18.83 mg/kg |
| MGS103 | 412.6 ± 46.34 mg/kg |
| MGS104 | 423.0 ± 35.21 mg/kg |
| MGS105 | 345.6 ± 24.86 mg/kg |

These compounds result from 75 to 95 times less toxic than physostigmine.

For all the substances examined the symptomatology of the toxic doses was essentially the same and consisted of muscular tremors, depressive phoenomena and finally death by asphyxial paralysis.

**"In vivo" anticholinesterase activity**

The derivatives under consideration were orally administered, suspended in 2% carboxy-methylcellulose, to Wistar male rats groups (average weight 250 g).

On the basis of the results obtained in preliminary trials, the doses administered were respectively: 0.2-0.4 mg/kg for physostigmine and 1.5 to 4.5 mg/kg for the substances to be studied.

The groups of rats treated were sacrificed after 20, 60, 120 minutes and their blood and their brains were immediately cooled to 0°C and maintained at this temperature during further treatments.

The acetylcholinesterase activity (AchE) was determined by means of the quick calorimetric method according to Ellmann et al (Biochem.Pharmacol. **7**, 88,1961), and expressed as percentage of enzymatic inhibition.

MGS101, MGS102, MGS103, MGS104, and MGS105 demonstrated to show a clear inhibitory dose-dependent and reversible both cerebral and serumal acetylcholinesterase activity, as one can see in the example reported for MGS101 in table 2 .

In order to obtain an anti-acetylcholinesterase activity of the same magnitudo order as that of physostigmine it was necessary to administer much higher doses than those used for physostigmine: on average 8-10 times higher.

The acetylcholinesterase activity duration of the same compounds in brain and in serum resulted considerably higher than that of physostigmine, as one can see from the example reported for MGS101 in table 3.

TABLE 2 Antiacetylcholinesterase activity in brain and in serum 20 minutes after the oral administration of various therapeutic doses both of physostigmine and of MGS101

| SUBSTANCE | DOSE mg/kg/os | No. OF RATS | INHIBITION % OF ACETYLCHOLINESTERASE AVERAGE VALUE ± EXPERIMENTAL ERROR | | $ED_{50}$ (mg/kg/os) | |
|---|---|---|---|---|---|---|
| | | | brain | serum | brain | serum |
| PHISOSTIGMINE | 0.2 | 5 | 16.72 ± 1.26 | 10.56 ± 1.45 | 0.37 (0.23 - 0.61) | 0.37 (0.24 - 0.58) |
| | 0.3 | 5 | 38.17 ± 9.21 | 33.32 ± 6.43 | | |
| | 0.4 | 5 | 52.30 ± 8.12 | 55.21 ± 11.95 | | |
| MGS 101 | 1.5 | 5 | 20.31 ± 3.12 | 17.4 ± 2.14 | 2.89 (1.83 - 4.59) | 2.89 (1.82 - 4.49) |
| | 2.5 | 5 | 41.60 ± 1.64 | 37.31 ± 4.86 | | |
| | 3.5 | 5 | 53.8 ± 11.25 | 60.44 ± 7.38 | | |
| | 4.5 | 5 | 76.42 ± 9.38 | 79.31 ± 3.81 | | |

TABLE 3 Antiacetylcholinesterase activity in rats* brain and serum after 20**, 60**, and 120 minutes from the oral administration of an equiactive dose of physostigmine and MGS101.

| SUBSTANCE | DOSE mg/kg/OS | INHIBITION % OF ACETYLCHOLINESTERASE (AchE) after....... AVERAGE VALUE ± EXPERIMENTAL ERROR | | | | | |
|---|---|---|---|---|---|---|---|
| | | 20 minutes | | 60 minutes | | 120 minutes | |
| | | brain | serum | brain | serum | brain | serum |
| PHISOSTIGMINE | 0.4 | 52.30 ± 9.12 | 55.11 ± 11.95 | 16.2 ± 4.61 | 11.4 ± 3.21 | 3.36 ± 1.45 | 2.16 ± 1.23 |
| MGS 101 | 3.5 | 53.8 ± 1.64 | 60.44 ±4.86 | 38.71 ±6.12 | 31.6 ± 4.25 | 24.21. ± 6.11 | 20.12 ± 2.40 |
| STATISTIC SIGNIFICATIVITY AMONG THE AVERAGE VALUES | | | | | | | |
| | p | NS | NS | <0.05 | <0.01 | <0.01 | < 0.01 |

* 5 animals per group

** The data reported for after 20 minutes are the same as those reported in table 2 ;those concerning 60 and 120 minutes relate to a different experiment.

## Spontaneous motility

The Dews method was used, modified according to Carminati (Arch. int. Pharmacodyn. et Thér 181, 68,1969).

MGS101,MGS102, MGS103, MGS104 and MGS105, orally administered caused in the mouse (Swiss weighing about 22-24 g: 10 animals per group) starting from the minimum doses of 4- 6 mg/kg, a significant decrease (on average -30%) of the spontaneous motility, the same effect was observed if physostigmine was orally administered at the dose of 0.6 mg/kg.

**Antagonist effect against the central stimulating effect of an anticholinergic substance**

As central stimulating anticholinergic substance, scopolamine was used, subcutaneously injected to groups of 10 SWISS mice (weighing about 22-26 g) at the dose of 1 mg/kg.

At this dose the substance provoked in all the animals a considerable increase (80-100%) of the spontaneous motility, studied according to the above mentioned method.

The preventive administration of the derivatives under consideration (30 minutes before the scopolamine administration), at the doses of 3-4 mg/kg/os, doses which are on their own not active for the motility, demonstrated to completely undo the hypermotor effect of scopolamine.

Under the same experimental conditions, physostigmine exhibited the same effect at the dose of 0.4 mg/kg/os.

**Electroencephalographic activity**

MGS101 was orally administered to a group of SWISS male mice (weighing about 28-30 g), which cortical electrodes were implanted to in the frontoparietal area according to the method described by Castellano and Oliverio (Brain res. 101, 317, 1976): the electrodes were connected to a polygraph (Transamerica: Instr., mod 8K42) in order to record the electroencephalogram.

The treatment with MGS101 at the dose of 6 mg/kg/os induced in the course of the successive 30 minutes, the formation of significant ecographic modifications, consisting in an increase of the amplitude and a decrease of the frequency of the electroencephalogram.

The same variations were observed after physostigmine was orally administered at the dose of 0.6 mg/kg.

**Effect on the duration of the curarization by succinylcholine**

At the dose of 0.1 mg intravenously administered, in the anesthetized rabbit and subjected to artificial respiration, physostigmine significantly prolonged the duration of the curare action of the succinylcholine. Said action can be demonstrated by recording the corneal reflex, the respiratory movements and the muscular contraction (Marotta and Carminati, Arch int. Pharmacodynamics et ther. 48, 255,1954). By using the same method (male rabbits of the tawny strain of Bourgogne; average weight 2.2 kg, under general anesthesy conditions by intravenous administration of ethyl urethane and subjected to artificial respiration) it was observed that the intravenous administration of 0.8 mg/kg of MGS101 and MGS102 induced the same anticholinesterase effect of physostigmine if compared with the curarization effect of succinylcholine.

**Pharmacokinetics**

In some preliminary tests single doses of MGS101 equal to 3-6 mg/kg were administered to Wistar male rats groups (weighing about 260 g: 5 animals per group).

The presence of said substance in plasma was detected by HPLC.

With the optimized method the detection limit was of 0.15 $\mu$g per ml of plasma.

After administration of 6 mg/kg/os, MGS 101 resulted present in plasma after 20 minutes at the concentration of 15.91 ± 4.21 $\mu$g/ml; the maximum peak of 33.81 ± 6.15 $\mu$g/ml was observed at 90'. The values declined very slowly (14.22 ± 3.12 $\mu$g/ml at the twelfth hour), up to the concentration of 4.31 ± 1.98 $\mu$g/ml, after 24 hours.

The above reported results demonstrate that the compounds under consideration are from 75 to 90 times less toxic, and only 8-10 times less active than physostigmine as antiacetylcholinesterase compounds.

Their therapeutic index is much more favourable than that of the comparison drug (physostigmine).

The antiacetylcholinesterase activity duration is much longer than that of physostigmine, presumably because of the slower elimination.

Pharmacological tests of the eseroline derivatives of formula (I) having the substituent W belonging to class B

**-Electrocardiographic activity**

Single doses of M001, M002, M003, M004, M005, M006, M007, M008 and M009 (in the range 0.4-7mg/kg), determine dose -dependence, reversible inhibition of acetylicholinesterase enzyme in the mouse brain.

The inhibitory activity of the above listed compounds lasts longer than that of physostigmine.

Even at higher doses these compounds, do not completely suppress the enzymatic activity of acetylcholinesterase.

**Antagonistic effect on the stimulating effect of scopolamine**

At the dose of 1 mg/kg, scopolamine produces an evident stimulating effect (100% increase in motor activity).

This effect is countered by the oral administration of 2.5 mg of the above mentioned compounds.

**Pharmacokinetics**

The kinetics of the products M001, M002, M003, M004, M005, M006, M007, M008, and M009 are studied in mice following oral (7mg/kg), and intramuscular (3.5 mg/kg) administration of these products.

The maximum peak (30-$\mu$g/ml) is found 1 hour after oral administration of these compounds.

Half-life of the products is about 15 hours.

High concentrations of said products are found in brain, and a similar behaviour is observed in heart.

Kinetics in liver indicate that these compounds pass through into the urine unchanged.

**Toxicity**

In the following table reported are LD50 values determined on mice for the listed compounds and compared with that of physostigmine administered by the same (intraperitoneal) route.

LD50 is evaluated on the grounds of the dose, in mg/kg, able to provoke death within 24 hours of 50% of the tested animals.

TABLE 4

| Compound | LD50 (mg/kg i.p.) |
|---|---|
| Physostigmine | 0.6 |
| M001 | >20<br><40 |
| M002 | >40<br><80 |
| M003 | >30<br><60 |
| M004 | >30<br><60 |
| M005 | >40<br><60 |
| M007 | >30<br><60 |
| M008 | >20<br><40 |
| M009 | >20<br><40 |
| M010 | >20<br><40 |

Pharmacological tests of the eseroline derivatives of formula (I) having the substituent W belonging to class C

**Electrocardiographic activity**

Single doses of ES001, ES002, ES003 (in the range 0.4-7mg/kg),determine a dose-dependence, reversible inhibition of the anticholinesterase activity, on the mouse brain.

The inhibitory activity of ES001, ES002, ES003 lasts longer than that of physostigmine.

ES001,ES002,ES003 even at higher doses, do not completely suppress the enzymatic activity.

**Antagonist effect on the stimulating effect of scopolamine**

At the dose of 1 mg/kg the scopolamine shows a clear stimulating effect (100% increase in motor activity).

This is countered by the oral administration of 2.5 mg of ES001, ES002, ES003.

**Pharmacokinetics**

The kinetics of the three products to be considered, is studied in mouse after oral and intramuscular administration of respectively 7 and 3.5 mg/kg respectively of the three compounds.

The maximum peak (30 µg/ml) is found 1 hour after oral administration.

The plasma bioavailability (AUC per os/AUC by intramuscular route) is about 30%, while the half-life is about 15 hours. High concentrations are found in brain, the brain bioactivity is 60%. A similar behaviour is observed in heart. The liver kinetics indicates that the three compounds pass through into the urine unchanged.

**-Analgesic activity**

The compounds of formula (I) according to the present invention show also analgesic activity.

This activity is demonstrated in mouse with 2-phenyl-1,4-benzoquinone test, which is a standard test for analgesic trials [Biol. Med., 95,729 (1957)].

The procedure followed represents a modification of the test developed by Haffner [ Dtsch.Med.Wschr.55, 731 (1929)].

**Toxicity**

In the following table reported are the LD50 values determined on mouse for the compounds listed and compared with the corresponding LD50 value for physostigmine, administered by the same (intraperitoneal) route.

TABLE 5

| Compound | LD50 (mg/kg i.p) |
|---|---|
| Physostigmine | 0.6 |
| ES001 | >20 <40 |
| ES002 | >40 <80 |
| ES003 | >40 <80 |
| ES004 | >10 <15 |
| ES005 | >15 <30 |
| ES007 | >40 <80 |
| ES008 | >20 <30 |

Pharmacological tests of the eseroline derivatives of formula (I) having the substituent W belonging to class D

The biological properties of some of the derivatives having the substituent W belonging to class D were compared with those of physostigmine.

Particularly the acute toxicity (LD50), the "in vivo" inhibition of cerebral and serumal acetylcholinesterase, the spontaneous motility, the antagonism against the central stimulating action of an anticholinergic agent (scopolamine), the electroencephalographic activity, and the effect on the curarization duration by succinylcholine.

Preliminary pharmacokinetics trials were also carried out.

**Acute toxicity**

The acute toxicity test was carried out on mice (Swiss strain of the average weight of 23 g (females) and 26 g (males) 5+5 animals per dose, period of observation 7 days).

The letal doses 50 (LD50) by oral route (suspension in 2 % carboxymethylcellulose) are reported hereinbelow:

TABLE 6

| LD50 per os in the mouse of physostigmine and of the eseroline derivatives having the substituent W belonging to class D | |
|---|---|
| Physostigmine | 4.12 ± 0.89 mg/kg |
| MGF001 | 461.44 ± 51.33 mg/kg |
| MGF002 | 436.72 ± 23.45 mg/kg |
| MGF005 | 428.42 ± 56.21 mg/kg |

These compounds result from 100 to 110 times less toxic than physostigmine.

For all the substances examined the symptomatology of the toxic doses was essentially the same and consisted of muscular tremors, depressive phoenomena and finally death by asphyxial paralysis.

**"In vivo" anticholinesterase activity**

The derivatives under consideration were orally administered, suspended in 2% carboxy-methylcellulose, to Wistar male rats groups (average weight 260 g).

On the base of the results obtained in preliminary trials, the doses administered were respectively: 0.2-0.4 mg/kg for physostigmine and 1-2 mg/kg for the substances to be studied.

The groups of rats treated were sacrificed after 20, 60, 120 minutes and their blood and their brains were immediately cooled to 0 ° C and maintained at this temperature during further treatments.

The acetylcholinesterase activity (AchE) was determined by means of the quick calorimetric method according to Ellmann et al (Biochem.Pharmacol. **7**, 88,1961), and expressed as percentage of enzymatic inhibition.

MGF001, MGF002, MGF005 demonstrated to show a clear inhibitory dose-dependent and reversible acetylcholinesterase activity, as one can see in the example reported for MGF001 in table 7 .

In order to obtain an acetylcholinesterase activity of the same magnitudo order as that of physostigmine were necessary much higher doses than those used for physostigmine: on average 5 times higher.

The acetylcholinesterase activity duration of the same compounds resulted considerably higher than that of physostigmine, as one can see from the example reported for MGF001 in the table 8.

TABLE 7- Antiacetylcholinesterase activity in brain and in serum 20 minutes after the oral administration of various therapeutic doses both of physostigmine and of MGF001

| SUBSTANCE | DOSE mg/kg/os | No. OF RATS | INHIBITION % OF ACETYLCHOLINESTERASE AVERAGE VALUE ± EXPERIMENTAL ERROR | | $ED_{50}$ (mg/kg/os) | |
|---|---|---|---|---|---|---|
| | | | brain | serum | brain | serum |
| PHISOSTIGMINE | 0.2 | 5 | 16.23 ± 3.41 | 17.12 ± 2.36 | 0.38 (0.22 - 0.64) | 0.39 (0.24 - 0.66) |
| | 0.3 | 5 | 29.22 ± 4.36 | 34.78 ± 6.83 | | |
| | 0.4 | 5 | 56.31 ± 7.35 | 51.28 ± 5.49 | | |
| MGF 001 | 1 | 5 | 14.48 ± 4.25 | 19.23 ± 2.28 | 1.83 (1.14 - 2.94) | 1.90 (1.13 - 3.20) |
| | 1.5 | 5 | 31.11 ± 3.41 | 36.58 ± 1.53 | | |
| | 2 | 5 | 59.3 ± 11.20 | 53.31 ± 7.47 | | |

TABLE 8 - Antiacetylcholinesterase activity in rats* brain and serum after 20**, 60**, and 120 minutes from the oral administration of an equiactive dose of physostigmine and MGF001.

| SUBSTANCE | DOSE mg/kg/OS | INHIBITION % OF ACETYLCHOLINESTERASE (AchE) after....... AVERAGE VALUE ± EXPERIMENTAL ERROR | | | | | |
|---|---|---|---|---|---|---|---|
| | | 20 minutes | | 60 minutes | | 120 minutes | |
| | | brain | serum | brain | serum | brain | serum |
| PHISOSTIGMINE | 0.4 | 56.31 ± 7.35 | 51.28 ± 5.49 | 27.21 ± 3.17 | 19.30 ± 2.46 | 3.61 ± 0.63 | 2.46 ± 1.20 |
| MGF 001 | 2 | 59.30 ±11.20 | 53.31 ± 7.47 | 46.38 ± 4.12 | 42.6 ± 5.28 | 25.45 ± 2.18 | 19.83 ± 4.27 |
| STATISTIC SIGNIFICATIVITY AMONG THE AVERAGE VALUES | | | | | | | |
| | p | NS | NS | <0.01 | <0.01 | <0.01 | <0.01 |

* 5 animals per group

** The data reported for after 20 minutes are the same as those reported in table 7; those concerning 60 and 120 minutes relate to a different experiment.

**Spontaneous motility**

The Dews method was used, modified according to Carminati (Arch. int. Pharmacodyn. et Thér 181, 68,1969).

MGF001,MGF002, and MGF005, orally administered caused in the mouse (Swiss weighing about 24-28

g:10 animals per group) at the doses of 2.5-5 mg/kg, a significant decrease (on average - 25%) of the spontaneous motility, the same effect was observed if physostigmine was orally administered at the dose of 0.6 mg/kg.

**Antagonist effect against the central stimulating effect of an anticholinergic substance**

As central stimulating anticholinergic substance, scopolamine was used, subcutaneously injected to groups of 10 SWISS mice (weghing about 22-26 g) at the dose of 1 mg/kg.

At this dose the substance provoked in all the animals a considerable increase (80-100%) of the spontaneous motility, studied according to the above mentioned method.

The preventive administration of the derivatives under consideration (30 minutes before the scopolamine administration), at the doses of 1.5-2 mg/kg/os, doses which are on their own not active for the motility, demonstrated to completely undo the hypermotor effect of scopolamine.

Under the same experimental conditions,physostigmine exhibited the same effect at the dose of 0.4 mg/kg/os.

**Electroencephalographic activity**

MGF001 was orally administered to a group of SWISS male mice (weighing about 27-30 g), which cortical electrodes were implanted to in the frontoparietal area according to the method described by Castellano and Oliverio (Brain res. **101**, 317, 1976): the electrodes were connected to a polygraph (Transamerica Instr., mod 8K42) in order to record the electroencephalogram.

The treatment with MGF001 at the dose of 3mg/kg/os induced in the course of the successive 30 minutes, the formation of significant ecographic modifications, consisting in an increase of the amplitude and a decrease of the frequency of the electroencephalogram.

The same variations were observed after physostigmine was orally administered at the dose of 0.6 mg/kg.

**Effect on the duration of the curarization by succinylcholine**

At the dose of 0.1 mg intravenously administered, in the anesthetized rabbit and subjected to artificial respiration, physostigmine significantly prolongs the duration of the curare action of the succinylcholine. Said action can be demonstrated by recording the corneal reflex, the respiratory movements and the muscular contraction (Marotta and Carminati, Arch int. Pharmacodynamics et ther. 48, 255,1954). By using the same method (male rabbits of the tawny strain of Bourgogne; average weight 2.2 kg under general anesthesy conditions by intravenous administration of ethyl urethane and subjected to artificial respiration) it was observed that the intravenous administration of 0.5 mg of MGF001 and MGF002 prolonged in the same way as physostigmine the curare effect of succinylcholine.

**Pharmacokinetics**

In preliminary tests single doses of MGF001 equal to 1-3 mg/kg were administered to Wistar male rats groups (weighing about 250 g: 5 animals per group).

The presence of said substance in plasma was detected by HPLC.

With the optimized method the detection limit was of 0.15 µg per ml of plasma.

After administration of 2mg/kg/os, MGF001 resulted present in plasma after 20 minutes at the concentration of 11.48 ± 2.23 µg/ml; the maximum peak of 47.60± 9.12 µg/ml was observed at 90'. After 120 minutes 28.41 ± 8.40 µg/ml and after 12 hours 10.64 ± 5.46 µg/ml.

The above reported results demonstrate that the compounds under consideration are from 95 to 110 times less toxic, and on average only 5 times less active than physostigmine as antiacetylcholinesterase compounds.

Their therapeutic index is much more favourable than that of the comparison drug (physostigmine).

The antiacetylcholinesterase activity duration is much longer than that of physostigmine, presumably because of the slower elimination.

Pharmacological tests of the eseroline derivatives of formula (I) having the substituent W belonging to class E

The biological properties of some of the derivatives having the substituent W belonging to class E were compared with those of physostigmine.

Particularly the acute toxicity (LD50), the "in vivo" inhibition of cerebral and serumal acetylcholinesterase, the spontaneous motility, the antagonism against the central stimulating action of an anticholinergic agent (scopolamine), the electroencephalographic activity.

Preliminary pharmacokinetics trials were also carried out.

**Acute toxicity**

The acute toxicity test was carried out on mice (Swiss strain of the average weight of 21 g (females) and 24 g (males) 5 + 5 animals per dose, period of observation 7 days).

The letal doses 50 (LD50) by oral route (suspension in 2 % carboxymethylcellulose) are reported herinbelow:

TABLE 9

| LD50 per os in the mouse of physostigmine and of the eseroline derivatives having the substituent W belonging to class A | |
|---|---|
| Physostigmine | 3.78 ± 0.86 mg/kg |
| MS001 | 51.40 ± 5.48 mg/kg |
| MS002 | 48.98 ± 6.31 mg/kg |
| MS007 | 64.26 ± 10.29 mg/kg |
| MS010 | 50.65 ± 5.48 mg/kg |
| MS011 | 63.31 ± 9.32 mg/kg |

These compounds result from 10 to 16 times less toxic than physostigmine.

For all the substances examined the symptomatology of the toxic doses was essentially the same and consisted of muscular tremors, depressive phoenomena and finally death by asphyxial paralysis.

**"In vivo" anticholinesterase activity**

The derivatives under consideration (MS001, MS002, MS007, MS010 and MS011 and physostigmine were orally administered, suspended in 2% carboxy-methylcellulose, to Wistar male rats groups (average weight 280 g).

On the basis of the results obtained in preliminary trials, the doses administered were respectively: 0.2-0.4 mg/kg for physostigmine and 0.1 to 0.25 mg/kg for the substances to be studied.

The groups of rats treated were sacrificed after 20, 60, 120 minutes and their blood and their brains were immediately cooled to 0 ° C and maintained at this temperature during further treatments.

The substances under consideration demonstrated to show a clear inhibitory dose-dependent and reversible both serumal and cerebral against acetylcholinesterase activity, as one can see in the example reported for MS001 in tables 10 and 11.

In order to obtain an antiacetylcholinesterase activity of the same magnitudo order as that of physostigmine it was necessary to administer doses equal to 1/2 the dose of physostigmine.

Both cerebral and serumal antiacetylcholinesterase activity duration of the same compounds resulted considerably longer than that of physostigmine, as one can see from the example reported for MS001 in table 11.

**TABLE 10 Antiacetylcholinesterase activity in brain and in serum 20 minutes after the oral administration of various therapeutic doses both of physostigmine and of MS001**

| SUBSTANCE | DOSE mg/kg/os | No. OF RATS | INHIBITION % OF ACETYLCHOLINESTERASE AVERAGE VALUE ± EXPERIMENTAL ERROR | | $ED_{50}$ (mg/kg/os) | |
|---|---|---|---|---|---|---|
| | | | brain | serum | brain | serum |
| PHISOSTIGMINE | 0.2 | 5 | 11.31 ± 6.36 | 14.63 ± 2.33 | 0.38 (0.24 - 0.60) | 0.37 (0.23 - 0.59) |
| | 0.3 | 5 | 33.67 ± 2.44 | 29.4 ± 3.41 | | |
| | 0.4 | 5 | 54.23 ± 8.17 | 59.4 ± 7.56 | | |
| MS 001 | 0.1 | 5 | 16.14 ± 9.43 | 21.42 ± 3.57 | 0.17 (0.12 - 0.23) | 0.18 (0.12 - 0.26) |
| | 0.15 | 5 | 38.52 ± 6.41 | 27.34 ± 6.22 | | |
| | 0.2 | 5 | 56.12 ± 7.21 | 52.36 ± 11.3 | | |
| | 0.25 | 5 | 83.40 ± 12.62 | 76.81 ± 8.38 | | |

**TABLE 11** Antiacetylcholinesterase activity in rats* brain and serum after 20**, 60**, and 120 minutes from the oral administration of an equiactive dose of physostigmine and MS001.

| SUBSTANCE | DOSE mg/kg/OS | INHIBITION % OF ACETYLCHOLINESTERASE (AchE) after....... AVERAGE VALUE ± EXPERIMENTAL ERROR | | | | | |
|---|---|---|---|---|---|---|---|
| | | 20 minutes | | 60 minutes | | 120 minutes | |
| | | brain | serum | brain | serum | brain | serum |
| PHISOSTIGMINE | 0.4 | 54.23 ± 8.17 | 59.4 ± 7.56 | 22.3 ± 5.12 | 18.41 ± 4.16 | 2.41 ± 1.33 | 3.44 ± 1.21 |
| MS 001 | 0.2 | 56.12 ± 7.21 | 52.36 ± 11.3 | 43.41 ± 3.25 | 36.22 ± 6.20 | 19.6 ± 3.41 | 23.12 ± 5.28 |
| STATISTIC SIGNIFICATIVITY AMONG THE AVERAGE VALUES | | | | | | | |
| P | | NS | NS | $<0.01$ | $<0.01$ | $<0.01$ | $<0.01$ |

* 5 animals per group

** The data reported for after 20 minutes are the same as those reported in table 10 ;those concerning 60 and 120 minutes relate to a different experiment.

## Spontaneous motility

The Dews method was used, modified according to Carminati (Arch. int. Pharmacodyn. et Thér 181, 68,1969).

MS001, MS002, MS007, MS 010 and MS011, orally administered caused in the mouse (Strain Swiss weighing about 22-24 g: 8 animals per group) at the doses of 0.25 mg/kg, a significant decrease (on average -25%) of the spontaneous motility, the same effect was observed if physostigmine was orally administered at the dose of 0.6 mg/kg.

**Antagonist effect against the central stimulating effect of an anticholinergic substance**

As central stimulating anticholinergic substance, scopolamine was used, subcutaneously injected to groups of 10 SWISS mice (weghing about 22-26 g) at the dose of 1 mg/kg.

At this dose the substance provoked a considerable increase (80-100%) of the spontaneous motility, studied according to the above mentioned method.

The preventive administration of the derivatives under consideration (30 minutes before the scopolamine administration), at the dose of 0.2 mg/kg/os, dose which is on its own not active for the motility, demonstrated to completely undo the hypermotor effect of scopolamine.

Under the same experimental conditions, physostigmine exhibited the same effect at the dose of 0.4 mg/kg/os.

**Electroencephalographic activity**

MS001 was orally administered to a group of SWISS male mice (weighing about 28-30 g), which cortical electrodes were implanted to in the frontoparietal area according to the method described by Castellano and Oliverio (Brain res. 101, 317, 1976): the electrodes were connected to a polygraph (Transamerica Instr., mod 8K42) in order to record the electroencephalogram.

The treatment with MS001 at the dose of 0.4 mg/kg/os induced in the course of the successive 30 minutes, the formation of significant ecographic modifications, consisting in an increase of the amplitude and a decrease of the frequency of the electroencephalogram.

The same variations were observed after physostigmine was orally administered at the dose of 0.6 mg/kg.

**Pharmacokinetics**

The presence of MS001 in plasma was detected by HPLC.

With the optimized method the detection limit was of 0.20 µg per ml of plasma.

In some preliminary tests conducted on Wistar male rats (weighing about 260 g: 5 animals per group) it was observed that after administration of 0.5 mg/kg/os, MS001 resulted present in plasma after 15 minutes (10.64 ± 6.26 µg/ml); the maximum peak of 41.24 ± 8.36 µg/ml was observed at 90'. After 120 minutes 32.12 ± 6.41 µg/ml and after twelve hours of 11.31 ± 3.18 µg/ml.

The above reported results demonstrate that the compounds under consideration are from 10 to 16 times less toxic, and 100 % more therapeutically active than physostigmine as antiacetylcholinesterase compounds.

Their therapeutic index is therefore much more favourable than that of the comparison drug (physostigmine).

The antiacetylcholinesterase activity duration is much longer than that of physostigmine, presumably because of the slower elimination.

<u>Pharmacological tests of the eseroline derivatives having the substituent W belonging to class F</u>

**Acute toxicity**

The toxicity is compared with that of physostigmine in mice (Swiss strain of both sexes weighing 22 g), using the oral administration .

| | | |
|---|---|---|
| LD50 | Physostigmine | 4.5 mg/kg |
| | MGR001 | 500 mg/kg |
| | MGR002 | 485 mg/kg |
| | MGR003 | 350 mg/kg |

**Electrocardiographic activity**

Single doses of MGR001, MGR002, MGR003 (0.4-7 mg/kg) determine dose-dependence and reversible inhibition of AChE (acetylcholinesterase) on the mouse brain.

The inhibitory activity of the three products lasts longer than that of physostigmine.
The three products even at higher doses do not completely suppress the enzymatic activity.

**Antagonistic effect on the stimulating effect of scopolamine.**

At the dose of 1 mg/kg the scopolamine produce a clear stimulating effect (100 % of motor activity increase). This is countered by the oral administration of 2.5 mg of the three products MGR001, MGR002 and MGR003.

**Pharmacokinetics**

The kinetics of MGR001, MGR002, and MGR003 is determined in rats after oral administration and intramuscular of respectively 7 and 3.5 mg/kg. The maximum peak of 30 µg/ml is found 1 hour after the oral administration.

Plasma bioavailability (AUC os/AUC i.m.) is about 30% and the half life about 15 hours.

High levels are found in the brain (7-9 times higher than those found in plasma) and bioactivity in brain is 60%. A similar behaviour is observed in heart.

Liver kinetics indicates that MGR001, MGR002, MGR004 pass through into the urine unchanged.

**Claims**

**1.** Eseroline derivatives of the general formula (I)

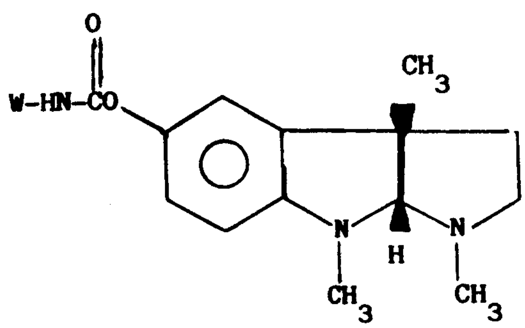

. x (HY)

wherein W is a substituent belonging to one of the following classes:

A)

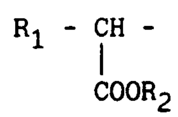

wherein $R_1$ is H, $-CH(CH_3)_2$, $-CH_2CH(CH_3)_2$,

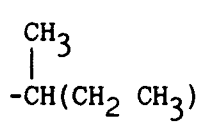

and

$R_2$ is H or a $C_1$-$C_{20}$ alkyl

B) - A $-COOR_3$

wherein A is selected from a $C_2$ - $C_8$ linear or branched alkyl radical optionally containing one or more ethylenic unsaturations, a $C_2$ - $C_8$ linear or branched ketoalkyl radical, a $C_2$-$C_8$ linear or branched hydroxyalkyl optionally esterified with an organic acid

$R_3$ is H or a $C_1$ - $C_{20}$ alkyl radical optionally containing one or more ethylenic unsaturations provided that when $R_3$ = H, A must be different from ketoalkyl;

C) $R_4$ OM;

wherein $R_4$ is H, or $CH_3$-$(L)_m$-CO- wherein L is a bivalent linear or branched $C_1$-$C_{20}$ alkyl radical, optionally containing one or more ethylenic unsaturations; m is 0 or 1

M is a $C_1$-$C_{20}$ bivalent linear or branched alkyl radical optionally substituted with an aryl radical; a bivalent alicyclic $C_3$-$C_7$ radical or is a bivalent aryl radical

D)

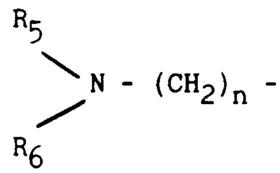

wherein $R_5$ and $R_6$ equal or different from eachother are selected from H, a $C_1$-$C_{20}$ alkyl radical, n is an integer comprised between 1 and 20

E)

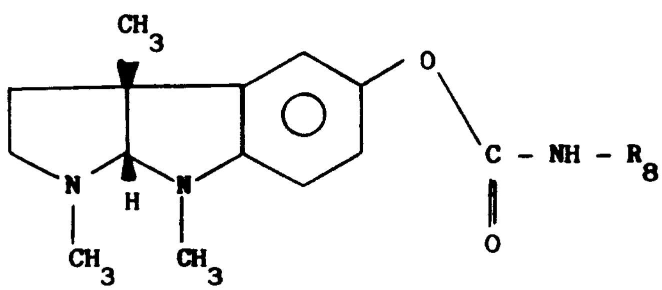

wherein $R_8$ is selected from the group consisting of:

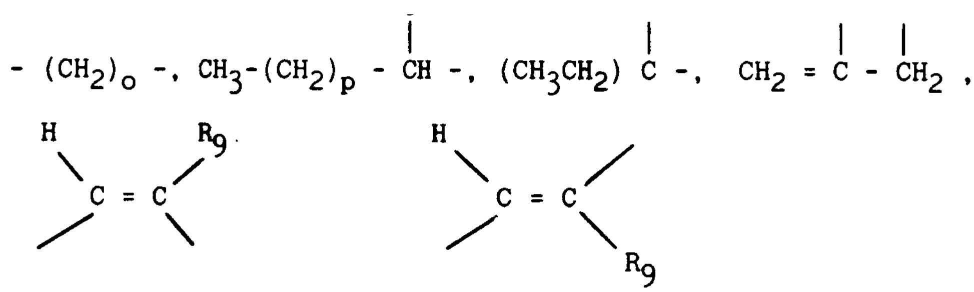

wherein o is an integer comprised between 1 and 8, p is an integer comprised between 1 and 3, $R_9$ is H or $CH_3$

F)

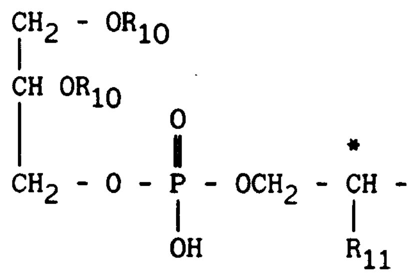

wherein $R_{11}$ is H or COOH, $R_{10}$ is H or a $C_2$-$C_{20}$ acyl radical, the carbon atom indicated with

asterisk when $R_{11}$ is COOH is an asymmetric carbon and is characterized by having L, D or (D,L) configuration
HY is a pharmaceutically acceptable organic or inorganic acid x may be 0, 1, 2 and 3 in case of monofunctional acids provided that x is 3 only when W is

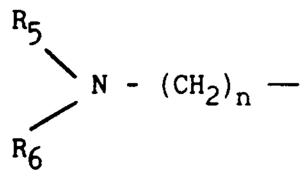

or 1/2 and 1/3 in case of respectively bisfunctional or trifunctional acids.

**2.** The eseroline derivative according to claim 1 wherein HY is selected from the group consisting of: tartaric acid, citric acid, salicylic acid, maleic acid, hydrochloric acid, phosphoric acid, sulforic acid.

**3.** The derivatives according to claim 1, having the substituent W belonging to class A and wherein:
a) $R_1$ is

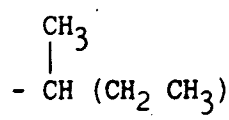

$R_2$ is H or n-heptyl
and $x = 0$
a') $R_1$ is

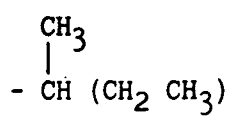

$R_2$ is H $x = 1$
and HY is a natural tartaric acid
a'') $R_1$ is $-CH_2\ CH\ (CH_3)_2$ $R_2$ is H and $x = 0$
a''') $R_1$ is $CH(CH_3)_2$ $R_2$ is H and $x = 0$
a$^{iv}$) $R_1$ and $R_2$ are H and x is 0

**4.** The eseroline derivative according to claim 1 having W which belongs to class B and wherein:
b') A is $-(CH_2)_2$ - and $R_3$ is H or $-C_9H_{19}$ $x = 0$
b'') A is $-(CH_2)_8-$ $R_3$ is H $x = 0$ or $= 1$ and in this case HY is tartaric acid
b''') A is $-CH_2CH(CH_3)$ and $R_3$ is H x is 0
b$^{iv}$) A is $-COCH_2-$, $R_3$ is $-CH_3$ and $x = 0$
b$^{v}$) A is $-CH(OH)CH_2-$ and $R_3$ is H and $x = 0$
b$^{vi}$) A is $-CH(CH_2OH)$ $R_3$ is H and $x = 0$
b$^{vii}$) A is $-CH_2\ (CHOH)\ CH_2$
$R_3$ is H
b$^{viii}$) A is $-CH_2\ CH_2CH(OH)CH_2$
$R_3$ is H and $x = 0$

**5.** The eseroline derivatives according to claim 1 having W which belongs to class C wherein
c) $R_4$ is H, M is

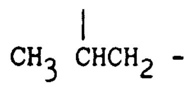

x = 0

c') $R_4$ is $CH_3(CH_2)_7 - CH = CH - (CH_2)_7$ COOH,

M is

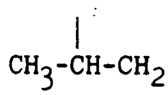

x = 0

c'') $R_4$ is H, M is

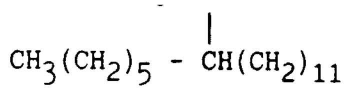

x = 0

c''') $R_4$ is H, M is

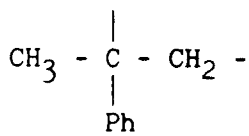

x = 0

c$^{iv}$) $R_4$ is H or $CH_3$ $(CH_2)_{16}$ - CO - M is

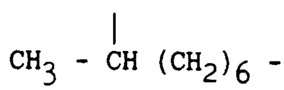

x = 0

c$^{v}$) $R_4$ is H,

M = 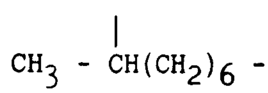

x = 0

c$^{vi}$) $R_4$ = H

M = 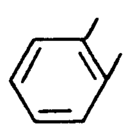

c$^{vii}$) $R_4$ = H

M = 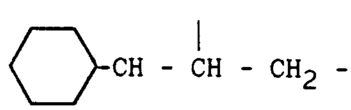

x = 0

$c^{viii}$) $R_4$ = H,

M = 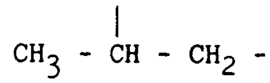

x = 1 HY = tartaric acid

**6.** The eseroline derivatives according to claim 1 belonging to class D wherein

d) $R_5$ = H, $R_6$ = methyl n is 7, x is 0

d') $R_5$ is H, $R_6$ is methyl, n is 5, x is 0

d'') $R_5$ and $R_6$ are n-heptyl, n is 4 , x is 0

d''') $R_5$ is H, $R_6$ is n-heptyl, n is 4, x is 0

$d^{iv}$) $R_5$ and $R_6$ are H, n is 7 and x is 0

$d^{v}$) $R_5$ is H, $R_6$ is methyl, n is 7, x is 1 , HA is salicylic acid.

**7.** The eseroline derivatives according to claim 1 belonging to class E wherein:

e) $R_8$ is $(-CH_2)_o$- and o is 1,2,3,4,5,6,7 or 8 and x is 0.

e') $R_8$ is $-(CH_2)_o-$ o is 8, x is = 2 and HY is salicylic acid

e'') $R_8$ is

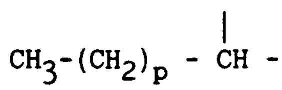

p is 3 and x is 0

e''') $R_8$ is

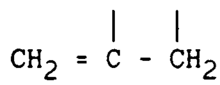

and x is 0

$e^{iv}$) $R_8$ is

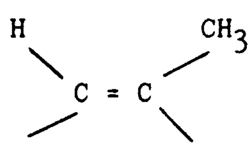

and x is 0

$e^{v}$) $R_8$ is

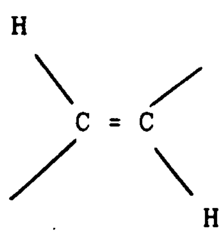

and x is 0

$e^{vi}$) $R_8$ is

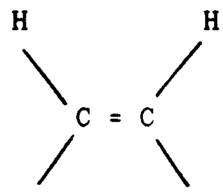

and x is 0

**8.** The eseroline derivatives according to claim 1 belonging to class F wherein
f) $R_{10}$ is $-COCH_3$, $R_{11}$ is H and x is 0
f') $R_{10}$ and $R_{11}$ are H and x is 0
f'') $R_{10}$ is $COCH_3$, $R_{11}$ is COOH and x is 0
f''') $R_{10}$ is $COCH_3$, $R_{11}$ is H, x is 1 and HY is natural tartaric acid

**9.** Therapeutical compositions containing as the active ingredient one or more derivatives of eseroline having the following formula

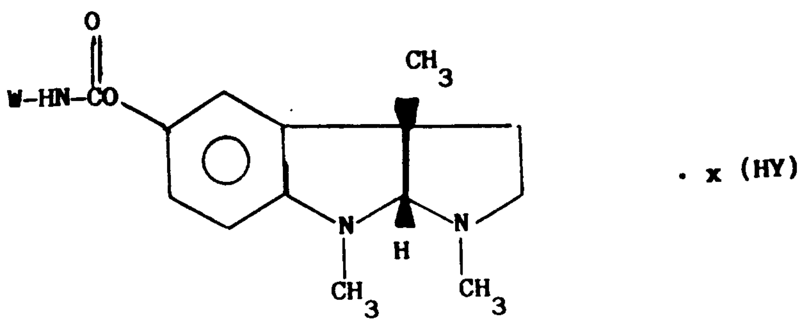

wherein W belongs to one of the following classes
A)

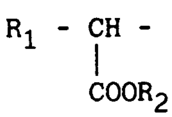

wherein $R_1$ is H, $-CH(CH_3)_2$, $-CH_2CH(CH_3)_2$,

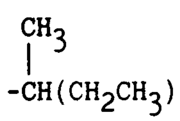

and $R_2$ is H or a $C_1$-$C_{20}$ alkyl
B) - A $-COOR_3$
wherein A is selected from a $C_2$ - $C_8$ linear or branched alkyl radical optionally containing one or more ethylenic unsaturations, a $C_2$ - $C_8$ linear or branched ketoalkyl radical, a $C_2$-$C_8$ linear or branched hydroxyalkyl optionally esterified with an organic acid;
$R_3$ is H or a $C_1$ - $C_{20}$ alkyl radical optionally containing one or more ethylenic unsaturations provided that when $R_3$ = H, A must be different from ketoalkyl;
C) $R_4OM$;
wherein $R_4$ is H, or $CH_3$ $(L)_m$-CO- wherein L is a bivalent linear or branched $C_1$-$C_{20}$ alkyl radical, optionally containing one or more ethylenic unsaturation, m is 0 or 1,
M is a $C_1$-$C_{20}$ bivalent linear or branched alkyl radical optionally substituted with an aryl radical; a

bivalent alicyclic $C_3$-$C_7$ radical, a bivalent aryl radical D)

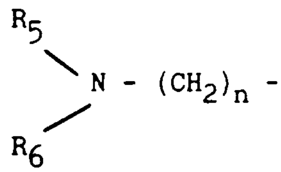

wherein $R_5$ and $R_6$ equal or different from eachother are selected from H, a $C_1$-$C_{20}$ alkyl radical, n is an integer comprised between 1 and 20

E)

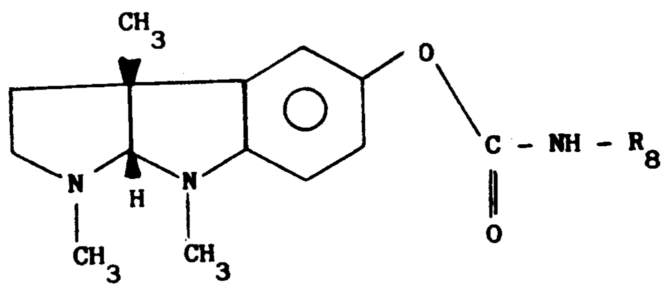

wherein $R_8$ is selected from the group consisting of:

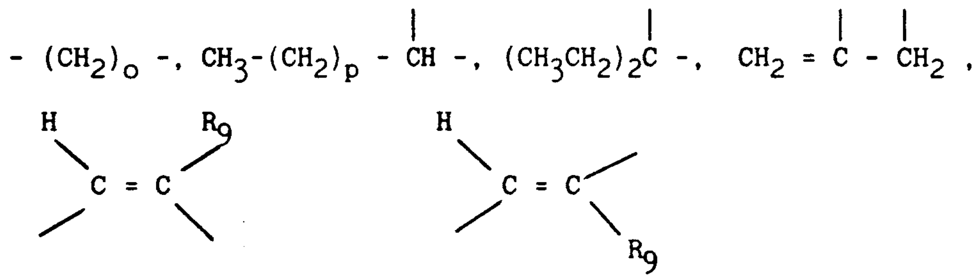

wherein o is an integer comprised between 1 and 8, p is an integer comprised between 1 and 3, $R_9$ is H or $CH_3$

F)

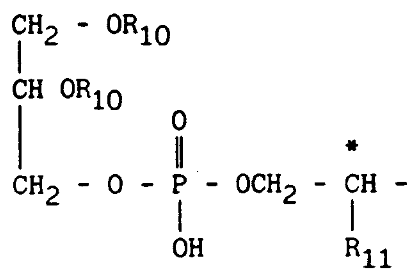

wherein $R_{11}$ is H or COOH, $R_{10}$ is H or a $C_2$-$C_{20}$ acyl radical, the carbon atom indicated with asterisk, when $R_{11}$ is COOH, is an asymmetric carbon and is characterized by having D, L or (D,L)-configuration

HY is a pharmaceutically acceptable organic or inorganic acid x may be 0, 1, 2 and 3 in case of monofunctional acids provided that x is 3 only when W is

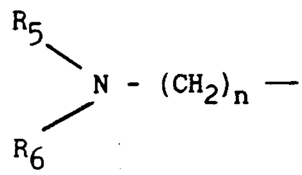

or is 1/2 and 1/3 in case of respectively bifunctional and trifunctional acids in combination with suitable excipients.

**10.** The therapeutical compositions according to claim 9 for the treatment of diseases associated with cholinergic disorders.

**11.** The therapeutical compositions according to claim 10 wherein the diseases associated with cholinergic disorders are Alzheimer disease, myasthenia gravis, glaucoma.

**12.** The therapeutical compositions according to claim 9 for the treatment of memory dysfunction caused by a decrease of the cholinergic function.

**13.** The therapeutical compositions according to claim 3 in the form of oral or parenteral compositions.

**14.** Use of the compound of general formula I

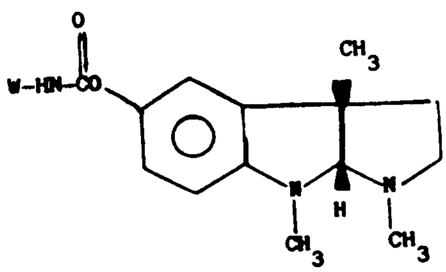

. x (HY)

wherein W: $R_4$ OM,
wherein $R_4$ is H or $CH_3$ $(L)_m$ - CO, L is bivalent linear or branched $C_1$-$C_{20}$ alkyl radical, optionally containing one or more ethylenic unaturations, m is 0 or 1, M is a $C_1$-$C_{20}$ bivalent linear or branched alkyl radical optionally substituted with an aryl radical, a bivalent alicyclic $C_3$-$C_7$ radical, or a bivalent aryl radical and HY is a pharmaceutically acceptable organic or inorganic acid and X may be 0, 1, or 2 in case of monofunctional acids or 1/2 and 1/3 in case of respectively bifunctional and trifunctional acids and as the active principle for the preparation of pharmaceutical composition having analgesic activity for the pain treatment.

**15.** A process for preparing the eseroline derivative of formula (I)

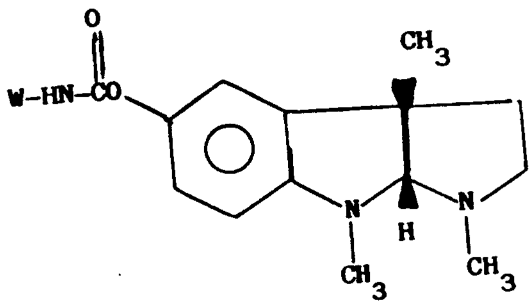

. x (HY)

wherein W is a substituent belonging to one of the following classes:
A)

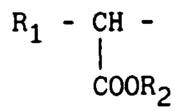

wherein $R_1$ is H, $-CH(CH_3)_2$, $-CH_2CH(CH_3)_2$,

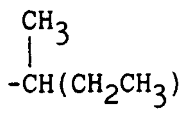

and $R_2$ is H or a $C_1$-$C_{20}$ alkyl

B) - A $-COOR_3$

wherein A is selected from a $C_2$ - $C_8$ linear or branched alkyl radical optionally containing one or more ethylenic unsaturation, a $C_2$ - $C_8$ linear or branched ketoalkyl radical, a $C_2$-$C_8$ linear or branched hydroxyalkyl optionally esterified with an organic acid

$R_3$ is H or a $C_1$ - $C_{20}$ alkyl radical optionally containing one or more ethylenic unsaturations provided that when $R_3$ is H, A must be different from ketoalkyl;

C) $R_4OM$;

wherein $R_4$ is H, or $CH_3$ $(L)_m$-CO- wherein L is a bivalent linear or branched $C_{1-20}$ alkyl radical, optionally containing one or more ethylenic unsaturations; m is 0 or 1

M is a $C_1$-$C_{20}$ bivalent linear or branched alkyl radical optionally substituted with an aryl radical; a bivalent alicyclic $C_3$-$C_7$ radical, a bivalent aryl radical

D)

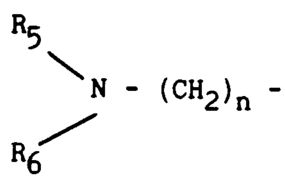

wherein $R_5$ and $R_6$ equal or different from eachother are selected from H, a $C_1$-$C_{20}$ alkyl radical, n is an integer comprised between 1 and 20

E)

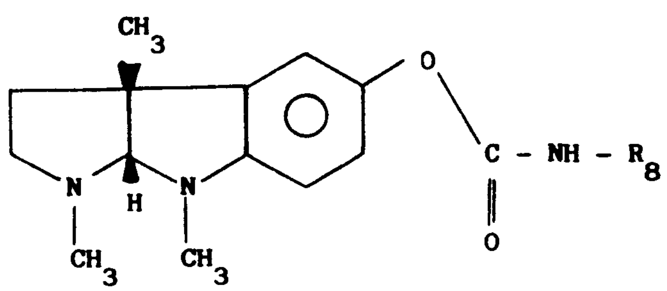

wherein $R_8$ is selected from the group consisting of:

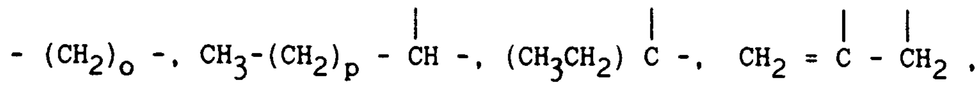

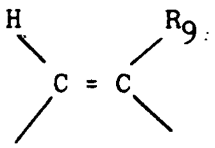
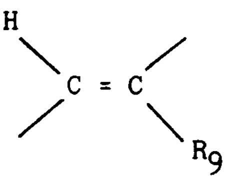

wherein o is an integer comprised between 1 and 8, p is an integer comprised between 1 and 3, $R_9$ H or $CH_3$

F)

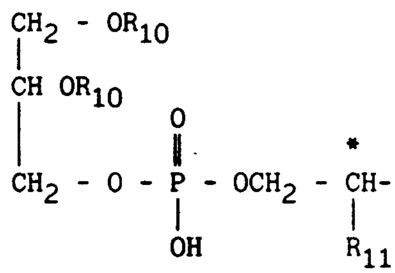

wherein $R_{11}$ is H or COOH, $R_{10}$ is H or a $C_2$-$C_{20}$ acyl radical, the carbon atom indicated with asterisk when $R_{11}$ is CO, is an asymmetric carbon and is characterized by having D, L or (D,L) configuration

HY is a pharmaceutically acceptable organic or inorganic acid x may be 0, 1, 2 and 3 in case of monofunctional acids provided that x is 3 only when w is

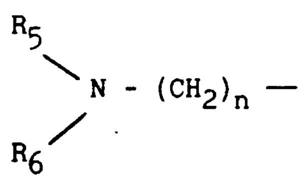

or 1/2 and 1/3 in case of respectively bifunctional and trifunctional acids comprising the following steps:

a) reacting the eseroline of formula (II)

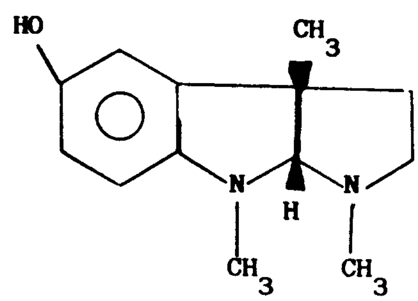

(II)

with the isocyanate of formula (III)

$W' - (N = C = O)_r$

wherein r is 1 or 2, W' is a substituent belonging to one of the following classes

A')

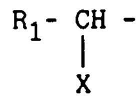

wherein $R_1$ has the above mentioned meaning
X is Cl or V wherein V is a carboxy protected group
B') $R'_3$OOC - A' -
wherein $R_3$ is $PhCH_2$ or = $R_3$ but different from H and A' = A but different from hydroxy- alkyl
C') MT,
wherein M, has the above mentioned meanings and T is a protecting group of the hydroxy function
and r is 1.
D')

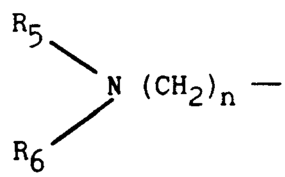

wherein $R'_5$ and $R'_6$ have the same meanings as $R_5$ and $R_6$ when they are both different from, when $R_5$ or $R_6$ is H, $R'_5$ or $R'_6$ is G wherein G is a protecting group of the amino function or they are = O in case in the final compound $R_5$ = $R_6$ = H and n has the above mentioned meanings.
E') W' = $R_8$ wherein $R_8$ has the above mentioned meanings
F')

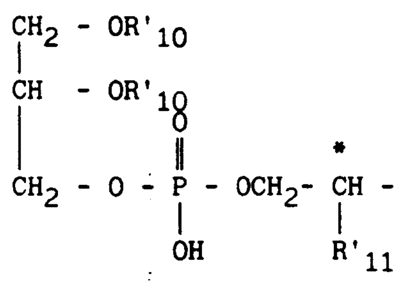

wherein $R_{11}$ = $R'_{11}$ in case this is = H and $R'_{11}$ = in wherein V is a protecting group of the -COOH function.

b) The compounds obtained in step (a) is treated with a stoichiometric amount of HY in a polar solvent to obtain the derivative of formula (I) having x = 0.

**16.** The process as claimed in claim 15 wherein step (a) is carried out in an aromatic solvent at room temperature for a time ranging from 1 to 6 hours and step (b) is carried out in absolute ethanol.

**17.** The process according to claim 15, wherein when in step (a) the isocyanate of formula (II) is used having r = 1 and W' belonging to class A') further comprising between step (a) and (b) the following steps:

i) when substituent X contained in W' = Cl, the compound obtained in step (a) is treated with magnesium, then with carbon dioxide and finally with an inorganic acid or

i') when X = V = -COO-Benzyl the compund coming from step (a) is treated with hydrogen at atmospheric pressure in a mixture of alcohol and an aprotic solvent in the presence of a

hydrogenation catalyst

ii) when compound (I) is to be obtained having $R_2$ different from H, the compound coming from step (a), (i), or (i') is treated in an organic solvent with a stoichiometric amount of alcohol $R_2OH$ wherein $R_2$ is as described above in the presence of a condensing agent and of a basic catalyst.

**18.** The process as claimed in claim 17 wherein step (i) is performed by using 1 to 1.5 moles of magnesium per mole, in an anhydrous aromatic solvent, at the boiling temperature of the solvent for a time ranging from 0.5 to 3 hours and the resulting reaction mixture is then treated with an at least stoichiometric amount of an inorganic acid or step (i') is carried out by using Pt/C as the catalyst in a mixture of toluene and ethanol and in step (ii) the solvent is tetrahydrofurane, the condensing agent is dicyclohexylcarbodiimide, the basic catalyst is dimethylaminopyridine.

**19.** The process according to claim 15 wherein when in step (a) the isocyanate of formula (III) is used having r = 1 and W' = Ph $PhCH_2OCO$-A'-

A' has the above mentioned meanings, the intermediate (IA) is obtained

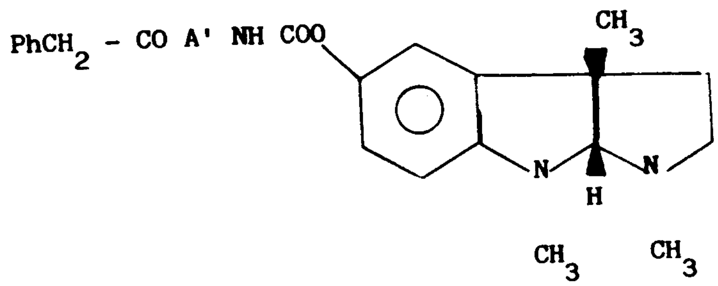

(IA)

and the process further comprises the following steps between step (a) and (b):

i) when the compound of formula (I) to be obtained presents $R_3$ = H, the carbamate of formula (IA) is treated with hydrogen at ambient temperature and at atmospheric pressure in a mixture of alcohol/aprotic solvent in ratio ranging from 1:9 to 1:0.5 respectively in the presence of a hydrogenating catalyst until it is completely converted into the derivative of formula (I) in which $R_3$ is H and A has the above mentioned meanings provided that it is different from ketoalkyl group;

ii) the product obtained in step (i) is esterified by treatment with an alcohol $R_3OH$ in which $R_3$ has the above mentioned meanings in the presence of a condensing agent and optionally a basic catalyst in aprotic solvent at a temperature ranging from 15 to 40 ° C, thereby obtaining the derivative of formula (I) wherein $R_3$, is different from H, and A is different from ketoalkyl.

**20.** The process as claimed in claim 19 characterized in that the solvent mixture of step (i) consists of a mixture ethanol/toluene in a volumetric ratio 1/1 and the catalyst is palladium carbon or $PtO_2$; and in step (ii) the reaction temperature is room temperature, the condensing agent is dicyclohexylcarbodiimide used in stoichiometric amount and the basic catalyst is diethylaminopyridine.

**21.** The process according to claim 15 wherein when in step (a) the isocyanate of formula (III) has r = 1 and W' belonging to class C' and it further comprises the following steps among step (a) and (b):

i) the protecting group T of the hydroxyl function is removed

ii) when the compound of formula (I) to be obtained presents $R_4 = CH_3 (L)_m - CO$ -, the compound obtained in step (i) is reacted with the corresponding acid of formula

$CH_3 (L_m) COOH$     (IV)

wherein L and m have the above mentioned meanings.

**22.** The process according to claim 21 wherein the protecting group T is-$OCH_2$ - Ph and step (i) is realized by carrying out a hydrogenation reaction in the presence of $PtO_2$ at atmospheric pressure in a mixture ethanol benzene at room temperature;

and step (ii) is carried out in the presence of stoichiometric amount of dicyclohexylcarbodiimide optionally in the presence of diethylaminopyridine in chloroform.

**23.** The process as claimed in claim 15 wherein in step (a) the isocyanate of formula (III) is used having W' belonging to class (E') and $r = 2$.

**24.** The process as claimed in claim 15 wherein when in step (a) the isocyanate of formula (III) is used having W' belonging to class (F') and $r = 1$ it further comprises between step (a) and (b) the following steps

(i) when the compound to be obtained of formula (I) presents $R_{10} = H$, it further comprises the following step among (a) and (b):

i) the product coming from step (a) is treated with an alkaline metal alkoxide in catalytic amounts per mole in alcohol, and the product obtained is recovered after neutralization with a mineral acid after the alkaline alkoxide, followed by neutralization with an inorganic base;

and /or

i') when in the compound to be obtained $R_{11}$ = COOH the product coming from step (a) having $R'_{11}$ = V = COOBenzyl, the compound is treated with hydrogen at atmospheric pressure in a mixture of an alcohol and an aprotic solvent, and in the presence of a hydrogenation catalyst.

**25.** The process according to claim 24 wherein in step (i) methanol or absolute ethanol is used as the solvent, sodium or potassium ethylate is used, as the alkaline metal alkoxide.

**26.** The process as claimed in claim 15, wherein when W' belongs to class D' it further comprises between step (a) and (b) the following step:

(i) when the compound of formula (I) to be obtained presents W having $R_5$ or $R_6$ = H, the product coming from step (a) in which W' brings $R'_5$ or $R'_6$ =-G, wherein -G has the above mentioned meanings, is treated with hydrogen at atmospheric pressure in the presence of a mixture of an aprotic solvent and alcohol in the presence of a hydrogenation catalyst;

or

(i') when the compound of formula (I) to be obtained presents W having $R_5 = R_6 = H$, the product coming from step (a) having W' wherein $R'_5 = R'_6$ = O is treated with $H_2$ by using as the catalyst Pt/C at atmospheric pressure.